# EUROPEAN PATENT APPLICATION

(11) **EP 4 252 757 A1**
(43) Date of publication of application: **04.10.2023**
(21) Application number: 21891939.7
(22) Date of filing: 11.11.2021
(51) Int. Cl.: A61K 31/7088, A61K 31/716, A61K 47/61, A61K 48/00, A61P 35/00, C12N 15/11

(54) **COMPLEX OF BETA-GLUCAN AND NUCLEIC ACID HAVING CONTROLLED PARTICLE SIZE**

(30) Priority: 12.11.2020 JP 2020189032
(71) Applicant: Daiichi Sankyo Company, Limited, Tokyo 103-8426 (JP)
(72) Inventor: ISHII, Ken, Ibaraki-shi, Osaka 567-0085 (JP); KUSAKABE, Takato, Ibaraki-shi, Osaka 567-0085 (JP); KASUYA, Yuji, Tokyo 103-8426 (JP); JONAI, Nao, Tokyo 103-8426 (JP); TAKESHITA, Fumihiko, Tokyo 103-8426 (JP); TANAKA, Isshin, Tokyo 134-8630 (JP); KUROSAWA, Emi, Tokyo 134-8630 (JP); SUZUKI, Kyosuke, Tokyo 134-8630 (JP); KOGA, Tetsufumi, Tokyo 103-8426 (JP); MIYAJI, Yoshihiro, Tokyo 134-8630 (JP)
(74) Representative: J A Kemp LLP
(86) International application number: PCT/JP2021/041490
(87) International publication number: WO 2022/102694

(57) **Abstract**

A complex of β(1→3) glucan and a nucleic acid, and having a controlled particle size is provided by the present invention. Furthermore, a complex of β(1→3) glucan and a nucleic acid, and having a controlled particle size is provided by the present invention.

## Description

### [Technical Field]

The present invention relates to a complex with a controlled particle size and formed by β-glucan and a nucleic acid.

### [Background Art]

CpG oligodeoxynucleotide (CpG ODN) is a single-stranded synthetic DNA of around 20 bases containing the immunostimulatory CpG motif. Toll-like receptor 9 (TLR9) has been identified as a host receptor for CpG ODN, and CpG ODN activates innate immunity via TLR9 and induces Type I interferons (hereinafter referred to as Type I IFNs) and inflammatory cytokines (Non Patent Literatures 1, and 2). TLR9 is expressed in plasmacytoid dendritic cells (hereinafter referred to as pDCs) and in the endosomes of B cells. CpG ODN taken up by pDCs and B cells interacts with TLR9 present in endosomes, and activates innate immunity (Non Patent Literature 2) .

There is a demand for the development of new immunotherapeutic agents for the prophylaxis or treatment of diseases such as infectious diseases, cancer, asthma, pollinosis, and the like, by using type I IFNs and inflammatory cytokines induced by CpG ODN (Non Patent Literatures 2 and 3). Pharmaceutical products containing CpG ODN that have already been put into practical use include a hepatitis B vaccine, Heplisav-B(R). Heplisav-B(R) is a prophylactic vaccine against hepatitis B virus (hereinafter referred to as HBV) infection, and CpG ODN is added as a vaccine adjuvant. Heplisav-B(R) has been recognized to have the possibility of showing higher efficacy than the existing aluminum adjuvant-added hepatitis B vaccine, Engerix-B(R). It has thus been suggested that CpG ODN may have higher vaccine adjuvant activity than the aluminum adjuvants that are most frequently used in vaccines (Non Patent Literature 4). Clinical trials using the anticancer activity of CpG ODN are also underway, and the development thereof as a therapeutic drug for metastatic melanoma is also underway (Non Patent Literature 5).

There are at least four types of CpG ODN, each of which has a different backbone, sequence, and immunostimulatory properties (Non Patent Literature 6). CpG ODNs, called Type A or Type D, typically contain one palindromic CpG motif with a phosphodiester (PO) backbone and phosphorothioate (PS) poly G tail, that activates pDCs to produce a large amount of IFN-α, but fails to induce pDC maturation and B cell activation (Non Patent Literatures 7, 8). CpG ODNs, called Type B or Type K, consist of a PS backbone, and many of them contain non-palindromic multiple CpG motifs and strongly activate B cells to produce inflammatory cytokines such as IL-6, activate pDCs to maturation, but do not induce IFN-α production (Non Patent Literatures 8 and 9). Many Type K CpG ODNs are described in Patent Literature 1. Type C and Type P which are other types of CpG ODN contain one and two palindromic CpG sequences, respectively, both of which can activate B cells like Type K and pDCs like Type D, though Type C CpG ODN induces weaker IFN-α production compared with Type P CpG ODN (Non Patent Literatures 10-12).

The Type D and Type P CpG ODNs form high-order structures, Hoogsteen base-pairing to form parallel quadruplex structures called G-tetrads, and Watson-Crick base-pairing between *cis-* and trans-palindromic portions, and these structures are required for robust IFN-α production by pDCs (Non Patent Literatures 12-14). Even though these higher-ordered structures are considered necessary for localization to early endosomes and signaling via TLR9, they are considered to cause polymorphism and aggregation of CpG ODNs as a disadvantage, thereby obstructing their clinical applications (Non Patent Literature 15). On the other hand, Type K and TYPE C CpG ODN impose no hurdles in production such as aggregation and are considered usable as immunotherapeutic agents and vaccine adjuvants for human use (Non Patent Literatures 16 and 17). As to Type K CpG ODN, moreover, results of enhanced immunogenicity of vaccines targeting infectious diseases and cancers have been reported in human clinical trials (Non Patent Literatures 6, 16).

Schizophylan (SPG), a soluble β(1→3) glucan derived from *Schizophyllum commune,* (hereinafter to be referred to as SPG) has been clinically used in Japan as an enhancer of radiotherapy in cervical carcinoma patients (Non Patent Literature 18). Similarly, lentinan (hereinafter LNT), a soluble β(1→3) glucan derived from shiitake mushroom, is a pharmaceutical product approved in 1985, and used in combination with a fluoropyrimidine medicament for inoperable and recurrent gastric cancer patients (Non Patent Literatures 19, 20). β(1→3) Glucan has been shown to form a complex with polydeoxyadenosine (hereinafter to be referred to as poly(dA)) as a triple helical structure (Non Patent Literature 21).

Patent Literatures 2 - 4 disclose use of a water-soluble complex of β(1→3) glucan including SPG and nucleic acid (gene), as a gene carrier. These documents describe that formation of the complex enhances an antisense activity of the gene and a resistance activity thereof against nuclease.

Patent Literature 5 discloses use of polysaccharides having a β(1→3) bond as a carrier (transfection agent) enhances the activity of an immunostimulating oligonucleotide having a CpG sequence, wherein a phosphodiester bond is substituted by a phosphorothioate bond or phosphorodithioate bond.

Patent Literature 6 discloses an immunostimulating complex consisting of an immunostimulating oligonucleotide and β(1→3) glucan having a long β(1→6) glucoside bond side chain.

It was demonstrated that mouse and humanized CpG ODN linked with poly dA having a phosphodiester bond at the 3'-end of CpG ODN complexed with SPG enhanced cytokine production and acted as an influenza vaccine adjuvant and a prophylactic or therapeutic agent for helper T cells type II (hereinafter Th2)-related diseases (Non Patent Literatures 22, 23, Patent Literature 7). In addition, it was shown that when the poly(dA) having phosphorothioate bond is linked to the 3'-end of CpG ODN, the complex formation increases by nearly 100% (Non Patent Literature 24).

When a complex of CpG ODN with 40 bases of dA linked to the 3'-end of Type K CpG ODN (K3) (hereinafter K3-dA40) and SPG (hereinafter the complex is referred to as K3-SPG) was allowed to act on human peripheral blood mononuclear cells (hereinafter to be referred to as hPBMCs), IFN-α production, which is not observed with K3 alone, was observed, and the level of IFN-α production was higher than that of Type D, Type P, Type C CpG ODNs under the conditions of lower concentrations of the nucleic acid treatment (Non Patent Literature 25). In addition, it was clarified that, in a mouse model, induction of IFN-α production by K3-SPG, and cytotoxic T cell (CTL) and helper T cell type 1 (hereinafter Th1) induction-enhancing activity when using K3-SPG as a vaccine adjuvant is dependent on Type I IFNs via TLR9 (Non Patent Literature 25). Furthermore, evaluation of the anticancer activity of K3-SPG alone using various mouse cancer transplantation models has revealed that the anticancer activity of intravenously administered K3-SPG is higher than that of K3 (Non Patent Literature 26). It has been suggested that the anticancer activity of K3-SPG in mouse cancer transplantation models is also dependent on type I IFNs, and may also be dependent on IL-12, which is important for CTL induction (Non Patent Literature 26).

Patent Literature 8 discloses a production method of an antigen/CpG oligonucleotide/β(1→3) glucan type ternary complex.

A complex formed by a β(1→3) glucan (BG) and CpG ODN nucleic acid (hereinafter to be referred to as BG-CpG) activates in vivo natural immunity, and has stronger physiological activities such as induction of inflammatory response than CpG ODN alone (Non Patent Literature 25). That is, BG-CpG is considered a potential immunotherapeutic agent for infectious diseases, cancer, and allergic diseases such as asthma and pollinosis, which are target diseases to which clinical application of CpG ODN alone is expected (Non Patent Literatures 2, 3, 25, 26).

Recently, the application of CpG ODN to cancer immunotherapy has attracted attention (Non Patent Literature 27). As an example of clinical application, nucleic acid preparations in which CpG ODN is encapsulated in virus-like nanoparticles (hereinafter referred to as VLPs) modified with CTL epitopes of melanoma cancer antigens induced melanoma cancer antigen-specific CTL in 14 of 22 melanoma patients in stages II to IV (Non Patent Literature 27). In non-clinical models, moreover, the anticancer activity of nanoparticulated CpG ODN has been reported against T-cell lymphoma, liver cancer, colon cancer, and glioma (Non Patent Literatures 28-31). These reports suggest the possibility that the particle size of the CpG ODN preparation is correlated with the anticancer activity, and BG-CpG in the form of nanoparticles is considered useful for cancer immunotherapy.

An index of important physiological activity of BG-CpG is, for example, the induction of IFN-α production, which is a Type I IFN (Non Patent Literature 25). In particular, the in vitro evaluation system that measures the induction level of IFN-α production using hPBMCs, which suggests the efficacy of BG-CpG in clinical practice, is superior in quantitativeness and data stability to the efficacy evaluation system that uses non-clinical cancer transplantation model in vivo (Non Patent Literatures 25 and 26). In addition, regarding in vitro efficacy evaluation in non-clinical mouse models, it is considered that anticancer activity in mouse transplantation model using living mice can be predicted by measuring IFN-α and IFN-γ production, which correlates with anticancer activity using living mice (Non Patent Literatures 25 and 26). On the other hand, it is considered necessary to clarify the cancer proliferation inhibitory activity of BG-CpG by evaluation using a mouse transplantation model (Non Patent Literature 26). Representative mouse cancer transplantation models include a B16 cancer subcutaneous transplantation model, which is a melanoma model, and a Pan-02 cancer peritoneal transplantation model, which is a peritoneal dissemination model of pancreatic cancer (Non Patent Literature 26).

As described above, the usefulness of BG-CpG in the pharmaceutical field, particularly cancer immunotherapy and vaccine adjuvant, has been confirmed. For practical application, it is desired to reduce the production cost by decreasing the dose associated with the improvement of BG-CpG activity, and to establish detailed production methods and quality evaluation methods. The present inventors took note of the particle size as one of the factors that determine the effectiveness of BG-CpG. As is well known to those of ordinary skilled in the art, it has been confirmed that BG-CpG used for this purpose has an average particle size of several tens of nm to several hundreds of nm in an aqueous medium (Patent Literatures 9-14). Pharmaceutical products with such size are called nanomedicine and are attracting attention as a pharmaceutical product form with properties and functions different from those of chemically synthesized pharmaceutical products or biopharmaceutical products manufactured using cell culture and the like. In recent years, guidelines relating to development and the like have been published one after another (Non Patent Literatures 32-34). These documents state that various properties need to be optimized in order to maximize the effectiveness of nanomedicine and achieve the quality that pharmaceutical products should possess. Among them, the particle size is considered to be one of the particularly important factors that determine the properties of nanomedicine.

Regarding the relationship between the particle size and the pharmacological activity of BG-CpG, there are the following reports. Patent Literature 10 discloses a range of 10 nm to 100 nm as a general average particle size and a range of 20 nm to 50 nm as a preferred average particle size for this use. It also discloses that the average particle size of BG-CpG (K3-SPG) that was actually tested was 30 nm. Patent Literature 11, which investigated the relationship between the particle size and pharmacological activity of BG-CpG with cell stimulation property in vitro as a basic index, discloses that a preferred inertia radius range for BG-CpG (complex of D35-dA40 as nucleic acid and SPG as β-glucan) is 20 nm to 200 nm. Specifically, as a result of comparison of the pharmacological activity of the complex having an inertia radius of 5 nm to 200 nm, an inertia radius of 20 nm to 150 nm is shown as a preferred range. More specifically, the following facts 1) to 3) are disclosed as the inertia radius dependency of the physiological activity possessed by the complex. 1) The complex with an inertia radius of 5 nm to 10 nm has a remarkably low physiological activity, 2) the complex with an inertia radius of 150 nm to 200 nm has a slightly low physiological activity, and 3) the complex with an inertia radius range of 20 nm to 150 nmm has no significant difference in activity.

Here, the low activity of complexes with a small inertia radius can be attributed to the fact that small complexes do not actually have sufficient complex formation efficiency, and that small molecules are not easily incorporated into cells. It is disclosed here that, even though different in the measurement principles, if the value obtained by multiplying the inertia radius by two is approximately the average particle size by conversion, the average particle size of the complex is preferably in the range of 40 nm to 300 nm, within which range the pharmacological activity of the complex does not vary remarkably. On the other hand, regarding the molecular weight of β-glucan as a component of BG-CpG and the particle size control method of BG-CpG, the above-mentioned Patent Literature 11 shows that, when the nucleic acid is D35-dA40, a series of BG-CpGs with different average particle sizes are obtained in the range of 10 nm or less to about 200 nm as the inertia radius, by complexing with SPGs having various molecular weights. Patent Literature 12 discloses a method for producing SPG having a molecular weight of 450,000 (as a trimer) but does not discuss an influence of the molecular weight of SPG on the particle size of the complex. Patent Literature 13 shows that β-glucan should have a weight-average molecular weight of 2000 or more, and that SPG with a molecular weight of 450,000 (as a trimer) was used as a constituent component of BG-CpG, but does not discuss an influence of the molecular weight of SPG on the particle size of the complex.

As described above, the relationship between the particle size and the pharmacological activity of BG-CpG has been studied in the average particle size range of several nm to several hundreds of nm, and the following are disclosed:
1) BG-CpG with an average particle size of 20 nm or less or 300 nm or more has lower activity than BG-CpG with an average particle size of 20 nm to 300 nm, and 2) even though the particle size dependency of the physiological activity of BG-CpG in the average particle size range of 20 nm to 300 nm is unclear, the range applied generally is 10 nm to 100 nm, and the preferred average particle size range is 20 nm to 50 nm. It is also found that BG-CpG with an average particle size of 30 nm is vigorously used as an anticancer agent or vaccine adjuvant.

### [Citation List]

### [Patent Literature]

[PTL 1]
   US 8,030,285 B2
[PTL 2]
   WO 01/034207 A1
[PTL 3]
   WO 02/072152 A1
[PTL 4]
   JP-A-2004-107272
[PTL 5]
   WO 2004/100965 A1
[PTL 6]
   JP-A-2007-70307
[PTL 7]
   JP-A-2008-100919
[PTL 8]
   JP-A-2010-174107
[PTL 9]
   WO 2016/103531
[PTL 10]
   WO 2015/041318
[PTL 11]
   WO 2016/098832
[PTL 12]
   WO 2004/100965
[PTL 13]
   WO 2001/034207
[PTL 14]
   JP-B-5605793

### [Non Patent Literature]

[NPL 1]
   Hemmi, H., et al. A Toll-like receptor recognizes bacterial DNA. Nature 408, 740-745 (2000).
[NPL 2]
   Krieg, A.M. Therapeutic potential of Toll-like receptor 9 activation. Nature reviews. Drug discovery 5, 471-484 (2006).
[NPL 3]
   Klinman, D.M. Immunotherapeutic uses of CpG oligodeoxynucleotides. Nature reviews. Immunology 4, 249-258 (2004).
[NPL 4]
   Schillie, S., et al. Recommendations of the Advisory Committee on Immunization Practices for Use of a Hepatitis B Vaccine with a Novel Adjuvant. MMWR Morb Mortal Wkly Rep. 2018 Apr 20; 67(15): 455-458.
[NPL 5]
   Ribas, A., et al. SD-101 in Combination with Pembrolizumab in Advanced Melanoma: Results of a Phase Ib, Multicenter Study. Cancer Discov. 2018 Oct; 8(10):1250-1257.
[NPL 6]
   Vollmer, J. & Krieg, A.M. Immunotherapeutic applications of CpG oligodeoxynucleotide TLR9 agonists. Advanced drug delivery reviews 61, 195-204 (2009).
[NPL 7]
   Krug, A., et al. Identification of CpG oligonucleotide sequences with high induction of IFN-alpha/beta in plasmacytoid dendritic cells. European journal of immunology 31, 2154-2163 (2001) .
[NPL 8]
   Verthelyi, D., Ishii, K.J., Gursel, M., Takeshita, F. & Klinman, D.M. Human peripheral blood cells differentially recognize and respond to two distinct CPG motifs. Journal of immunology 166, 2372-2377 (2001).
[NPL 9]
   Hartmann, G. & Krieg, A.M. Mechanism and function of a newly identified CpG DNA motif in human primary B cells. Journal of immunology 164, 944-953 (2000).
[NPL 10]
   Hartmann, G., et al. Rational design of new CpG oligonucleotides that combine B cell activation with high IFN-alpha induction in plasmacytoid dendritic cells. European journal of immunology 33, 1633-1641 (2003).
[NPL 11]
   Marshall, J.D., et al. Identification of a novel CpG DNA class and motif that optimally stimulate B cell and plasmacytoid dendritic cell functions. Journal of leukocyte biology 73, 781-792 (2003).
[NPL 12]
   Samulowitz, U., et al. A novel class of immune-stimulatory CpG oligodeoxynucleotides unifies high potency in Type I interferon induction with preferred structural properties. Oligonucleotides 20, 93-101 (2010).
[NPL 13]
   Kerkmann, M., et al. Spontaneous formation of nucleic acid-based nanoparticles is responsible for high interferon-alpha induction by CpG-A in plasmacytoid dendritic cells. The Journal of biological chemistry 280, 8086-8093 (2005).
[NPL 14]
   Klein, D.C., Latz, E., Espevik, T. & Stokke, B.T. Higher order structure of short immunostimulatory oligonucleotides studied by atomicforce microscopy. Ultramicroscopy 110, 689-693 (2010).
[NPL 15]
   Puig, M., et al. Use of thermolytic protective groups to prevent G-tetrad formation in CpG ODN Type D: structural studies and immunomodulatory activity in primates. Nucleic acids research 34, 6488-6495 (2006).
[NPL 16]
   Bode, C., Zhao, G., Steinhagen, F., Kinjo, T. & Klinman, D.M. CpG DNA as a vaccine adjuvant. Expert review of vaccines 10, 499-511 (2011).
[NPL 17]
   McHutchison, J.G., et al. Phase 1B, randomized, double-blind, dose-escalation trial of CPG 10101 in patients with chronic hepatitis C virus. Hepatology 46, 1341-1349 (2007).
[NPL 18]
   Okamura, K., et al. Clinical evaluation of schizophyllan combined with irradiation in patients with cervical cancer. A randomized controlled study. Cancer 58, 865-872 (1986).
[NPL 19]
   Oba, K.; Kobayashi, M.; Matsui, T.; Kodera, Y.; Sakamoto, J. Individual patient based meta-analysis of lentinan for unresectable/recurrent gastric cancer. Anticancer Res., 2009, 29, 2739-2746.
[NPL 20]
   Nakano, H.; Namatame, K.; Nemoto, H.; Motohashi, H.; Nishiyama, K.; Kumada, K. A multi-institutional prospective study of lentinan in advanced gastric cancer patients with unresectable and recurrent diseases: Effect on prolongation of survival and improvement of quality of life. Hepato-Gastroenterol., 1999, 46, 2662-2668.
[NPL 21]
   Sakurai, K., Mizu, M. & Shinkai, S. Polysaccharide-polynucleotide complexes. 2. Complementary polynucleotide mimic behavior of the natural polysaccharide schizophyllan in the macromolecular complex with single-stranded RNA and DNA. Biomacromolecules 2, 641-650 (2001) .
[NPL 22]
   Shimada, N., et al. A polysaccharide carrier to effectively deliver native phosphodiester CpG DNA to antigen-presenting cells. Bioconjugate chemistry 18, 1280-1286 (2007).
[NPL 23]
   Koyama, S., et al. Plasmacytoid dendritic cells delineate immunogenicity of influenza vaccine subtypes. Science translational medicine 2, 25ra24 (2010).
[NPL 24]
   Minari, J., et al. Enhanced cytokine secretion from primary macrophages due to Dectin-1 mediated uptake of CpG DNA/beta-1,3-glucan complex. Bioconjugate chemistry 22, 9-15 (2011).
[NPL 25]
   Kobiyama, K., et al. Nonagonistic Dectin-1 ligand transforms CpG into a multitask nanoparticulate TLR9 agonist. PNAS 111, 3086-3091 (2014).
[NPL 26]
   Kitahata, Y., et al. Circulating nano-particulate TLR9 agonist scouts out tumor microenvironment to release immunogenic dead tumor cells. Oncotarget 7, 48860-48869 (2016).
[NPL 27]
   Speiser DE, Schwarz K, Baumgaertner P, et al. Memory and effector CD8 T-cell responses after nanoparticle vaccination of melanoma patients. J Immunother. 2010; 33(8):848-858.
[NPL 28]
   Nikitczuk KP, Schloss RS, Yarmush ML, Lattime EC. PLGA-polymer encapsulating tumor antigen and CpG DNA administered into the tumor microenvironment elicits a systemic antigen-specific IFN-γ response and enhances survival. J Caner Ther. 2013; 4(1):280-290.
[NPL 29]
   Kwon S, Kim D, Park B, et al. Induction of immunological memory response by vaccination with TM4SF5 epitope-CpG-DNA-liposome complex in a mouse hepatocellular carcinoma model. Oncology Rep. 2013; 29(2):735-740.
[NPL 30]
   Kwon S, Kim Y-E, Park J-A, Kim D-S, Kwon H-J, Lee Y. Therapeutic effect of a TM4SF5-specific peptide vaccine against colon cancer in a mouse model. BMB Rep. 2014; 47(4):215-220.
[NPL 31]
   Zhao D, Alizadeh D, Zhang L, et al. Carbon nanotubes enhance CpG uptake and potentiate antiglioma immunity. Clin Cancer Res. 2011; 17(4):771-782.
[NPL 32]
   "On publication, etc. of Joint MHLW/EMA reflection paper on the development of block copolymer micelle medicinal products", Ministry of Health, Labour and Welfare, Japan, PFSB/ELD Notification No. 0110-1, January 10, 2014.
[NPL 33]
   "On Reflection paper on nucleic acids (siRNA)-loaded nanotechnology-based drug products", Evaluation and Licensing Division, Pharmaceutical Safety and Environmental Health Bureau, Ministry of Health, Labour and Welfare, Japan, March 28, 2016.
[NPL 34]
   "On Guideline for the Development of Liposome Drug Products", Evaluation and Licensing Division, Pharmaceutical Safety and Environmental Health Bureau, Ministry of Health, Labour and Welfare, Japan, March 28, 2016.

### [Summary of Invention]

### [Technical Problem]

BG-CpG has a characteristic structure in which β-glucan and a nucleic acid are loosely assembled through non-covalent bonds, but the findings relating to the relationship between the particle size and the pharmacological activity of BG-CpG are still insufficient. Therefore, in order to obtain a pharmaceutical product composed of a complex of β-glucan and nucleic acid such as BG-CpG, it is necessary to investigate in detail an influence of particle size on pharmacological activity. It is an object of the present invention to find a conventionally-unknown preferred range of particle size for a complex of β-glucan and a nucleic acid. Furthermore, it is also an object of the present invention to establish a highly robust production method and analytical method for producing a complex of β-glucan and nucleic acid such as BG-CpG, in which the particle size is controlled within a preferred range.

### [Solution to Problem]

The present inventors have conducted intensive studies and produced a series of BG-CpG with an average particle size that varies in steps. As a result, the present inventors have found that BG-CpG with a strictly controlled average particle size in the range of about 20 nm to 180 nm, preferably about 20 nm to 130 nm or about 40 nm to 180 nm, more preferably about 80 nm to 130 nm or about 130 nm to 180 nm, can be produced. They have investigated the anticancer activity of BG-CpG with an average particle size in the range of 20 nm to 130 nm or 40 nm to 180 nm, and found that the activity becomes greater as the average particle size becomes larger.

Accordingly, the present invention includes the following.
[1] A complex of β(1→3) glucan and a nucleic acid, having a controlled particle size.
[2] The complex of [1], wherein the average particle size is 80 nm to 130 nm.
[3] The complex of [1], wherein the average particle size is 130 nm to 180 nm.
[4] The complex of [1] or [2], wherein the aforementioned β(1→3) glucan is schizophyllan.
[5] The complex of [1] or [3], wherein the aforementioned β(1→3) glucan is lentinan.
[6] The complex of any one of [1] to [5], wherein the aforementioned nucleic acid is an oligonucleotide comprising a Type K CpG oligonucleotide.
[7] The complex of any one of [1] to [6], wherein the aforementioned nucleic acid is a nucleic acid with a polydeoxyadenosine linked to the 3'-end of a Type K CpG oligonucleotide.
[8] The complex of any one of [1] to [7], wherein the aforementioned nucleic acid is an oligodeoxynucleotide comprising a humanized Type K CpG oligodeoxynucleotide consisting of the nucleotide sequence shown in SEQ ID NO: 1 and a polydeoxyadenylic acid, the polydeoxyadenosine is linked to the 3'-end of the humanized Type K CpG oligodeoxynucleotide, and phosphodiester bonds in the oligodeoxynucleotide are partly or entirely substituted by phosphorothioate bonds.
[9] A pharmaceutical composition comprising the complex of any one of [1] to [8].
[10] The pharmaceutical composition of [9], which is for the prophylaxis or treatment of a viral infectious disease, cancer, an allergic disease, or intracellular parasitic protozoan or bacterial infection.
[11] The pharmaceutical composition of [10], which is for the prophylaxis or treatment of a viral infectious disease.
[12] The pharmaceutical composition of [11], wherein the viral infectious disease is an RS virus or influenza virus infectious disease.
[13] An immunostimulating agent comprising the complex of any one of [1] to [8].
[14] The immunostimulating agent of [13], which is a vaccine adjuvant.
[15] A pharmaceutical composition comprising
   (a) the complex of any one of [1] to [8] or the immunostimulating agent of [13] or [14], and
   (b) an antigen.
[16] The composition of [15], which is for inducing an immune response to the antigen.
[17] The composition of [16], wherein the antigen is derived from a pathogen.
[18] The composition of [17], which is for the prophylaxis or treatment of a pathogen infectious disease.
[19] The composition of [18], wherein the pathogen is a virus.
[20] The composition of [19], wherein the virus is an RS virus or an influenza virus.
[21] The composition of [16], wherein the antigen is derived from cancer.
[22] The composition of [21], which is for the prophylaxis or treatment of cancer.
[23] A pharmaceutical composition comprising
   (a) the complex of any one of [1] to [8], or the pharmaceutical composition of any one of [9], [10], [15], [16], [21], and [22], and
   (b) an anticancer agent.
[24] The composition of [23], wherein the anticancer agent is an immune checkpoint inhibitor.
[25] The composition of [24], wherein the immune checkpoint inhibitor is any of a PD1 inhibitor, a PDL-1 inhibitor, and a CTLA-4 inhibitor.
[26] A method for producing the complex of any one of [1] to [8], utilizing the molecular weight property of β(1→3) glucan.
[27] A method for treating or preventing viral infectious disease, cancer, an allergic disease, or intracellular parasitic protozoan or bacterial infection, comprising administering an effective amount of a pharmaceutical composition comprising the pharmaceutical composition of [9] to a test subject.
[28] The method of [27], wherein the viral infectious disease is an RS virus or influenza virus infectious disease.
[29] A method for treating or preventing viral infectious disease, cancer, an allergic disease, or intracellular parasitic protozoan or bacterial infection, comprising administering an effective amount of a pharmaceutical composition comprising the pharmaceutical composition of [15] to a test subject.
[30] The method of [29], wherein the viral infectious disease is an RS virus or influenza virus infectious disease.
[31] The method of [27] or [29] which is used in combination with other anticancer agent.
[32] The method of [31], wherein the anticancer agent is an immune checkpoint inhibitor.
[33] The method of [32], wherein the immune checkpoint inhibitor is any of a PD1 inhibitor, a PDL-1 inhibitor, and a CTLA-4 inhibitor.

### [Advantageous Effects of Invention]

According to the present invention, BG-CpG with a strictly controlled average particle size in the range of 20 nm to 180 nm, preferably 20 nm to 130 nm or 40 nm to 180 nm, more preferably 80 nm to 130 nm or 130 nm to 180 nm, is provided by selecting or controlling the molecular weight property of β-glucan by a highly practical production method and production conditions. Therefore, an optimal particle size can be selected for a therapeutic drug or a prophylactic drug for cancer immunotherapy, or as one of the constituent components of these. The BG-CpG of the present invention may be applicable to diseases that are difficult to treat or prevent with conventional BG-CpG, because it exhibits remarkable effectiveness at a lower dose than conventional BG-CpG.

### [Brief Description of Drawings]

[Fig. 1]

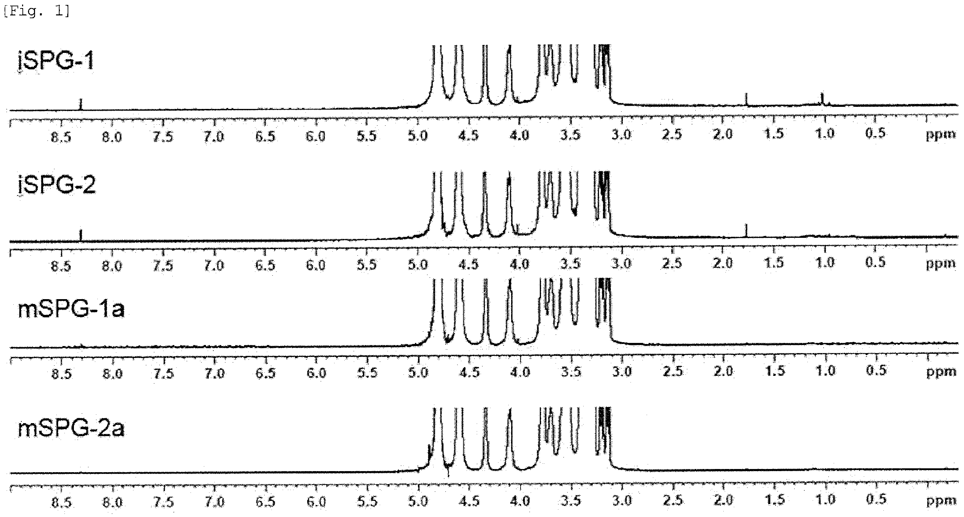

   Fig. 1 is a chart of ¹H-NMR spectra of the produced mSPG and SPG standard samples.
[Fig. 2]

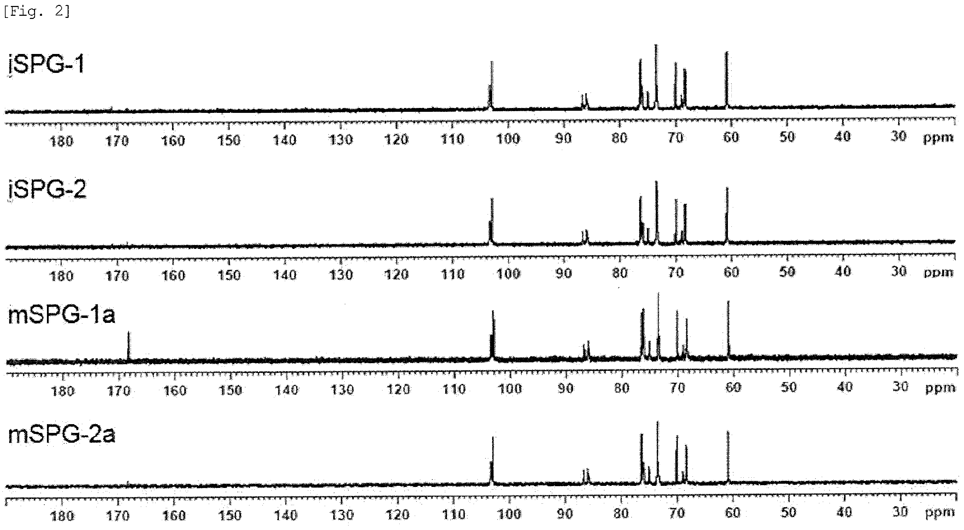

   Fig. 2 is a chart of ¹³C-NMR spectra of the produced mSPG and SPG standard samples.
[Fig. 3]

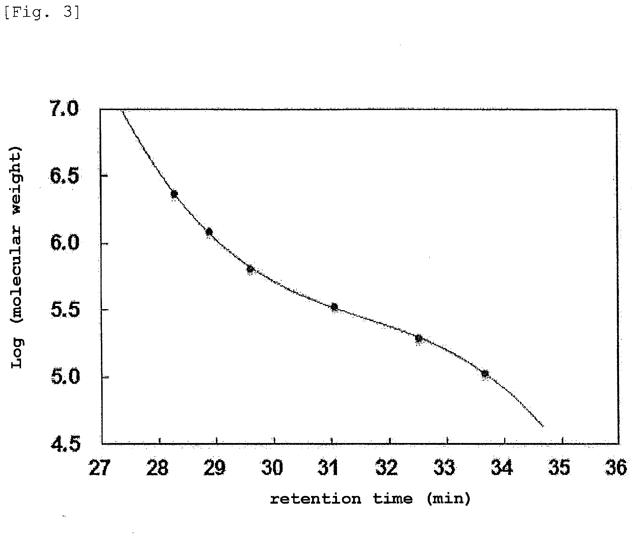

   Fig. 3 shows a molecular weight calibration curve of pullulan.
[Fig. 4]

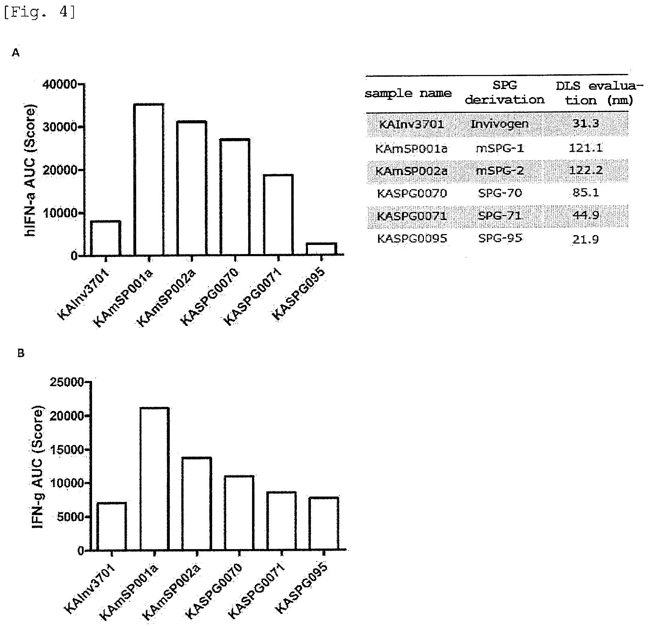

   Fig. 4A is a graph showing the evaluation results of the potency of K3-SPG to induce IFN-α production from human PBMC. Fig. 4A shows AUC values of IFN-α production levels when hPBMCs were stimulated with K3-SPG at concentrations of 0, 0.75, 2, 6, and 20 µg/mL. Fig. 4B is a graph showing the results of evaluating the potency to induce IFN-γ production from mouse spleen cells. Fig. 4B shows AUC values of IFN-γ induction levels when mouse spleen cells were stimulated with K3-SPG at concentrations of 0, 0.75, 2, 6, and 20 µg/mL. The lot numbers of the test substances used in Fig. 4 are as follows:
   ·KAInv3701:20170613-01
   ·KAmSP001a:20170613-02
   ·KAmSP002a:20170613-03
   ·KASPG0070:20170613-04
   ·KASPG0071:20170613-05
   ·KASPG0095:20170926-07.
   The evaluation results of DLS (nm) of the samples of respective lot numbers are shown on the right of the graph in Fig. 4A.
[Fig. 5]

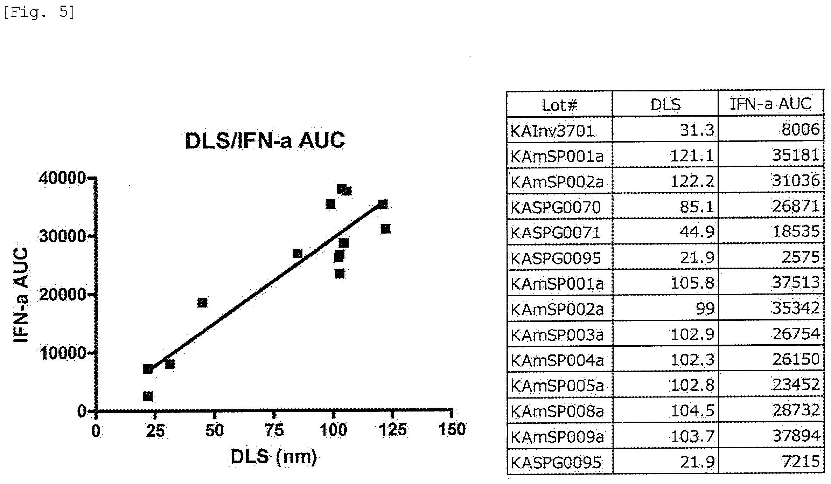

   Fig. 5 is a diagram showing the results of evaluation by Spearman's test of the correlation between the AUC values of IFN-α production levels when hPBMCs were stimulated with K3-SPG at CpG ODN concentrations of 0, 0.75, 2, 6, 20 and the average particle size using DLS.
[Fig. 6]

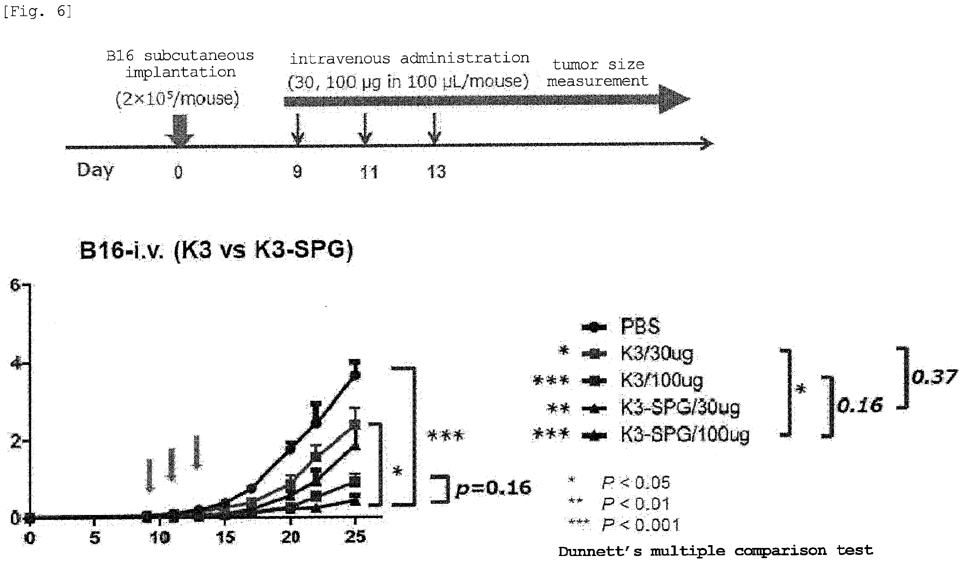

   Fig. 6 shows an in vivo evaluation of the pharmacological activity of K3-SPG. The upper part of Fig. 6 shows the protocol of the in vivo evaluation experiment. The lower part of Fig. 6 is a graph showing the measurement results of the tumor size of K3-SPG, K3, and PBS (control) over time according to the protocol. In the graph in the lower part of Fig. 6, the horizontal axis indicates the number of days after subcutaneous implantation of B16, and the vertical axis indicates the tumor size of the K3-SPG, K3, and PBS-administration groups.
[Fig. 7]

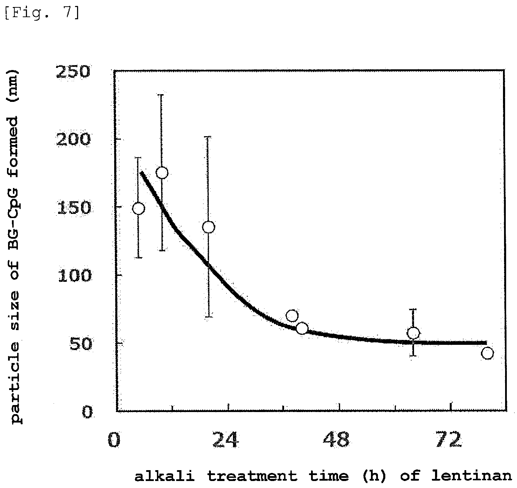

   Fig. 7 shows the relationship between the alkali treatment time and the average particle size of BG-CpG containing lentinan as a component, when lentinan was treated with alkali under predetermined conditions.
[Fig. 8]

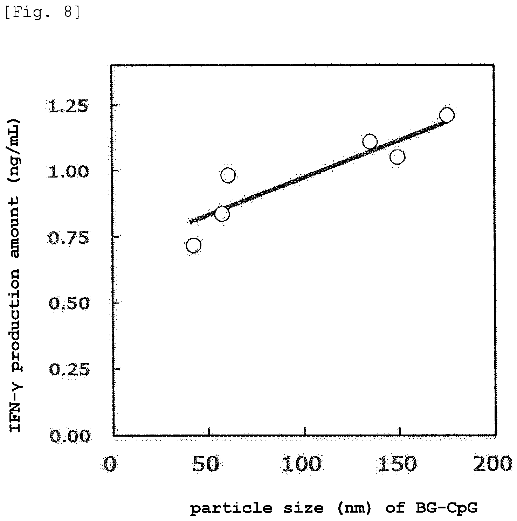

   Fig. 8 is a graph showing the evaluation results of the potency of K3-LNT with various average particle sizes to induce IFN-γ production from mouse spleen cells. Total IFN-γ induction levels when mouse spleen cells were stimulated with K3-LNT at concentrations of 2 and 10 µg/mL are shown.
[Fig. 9]

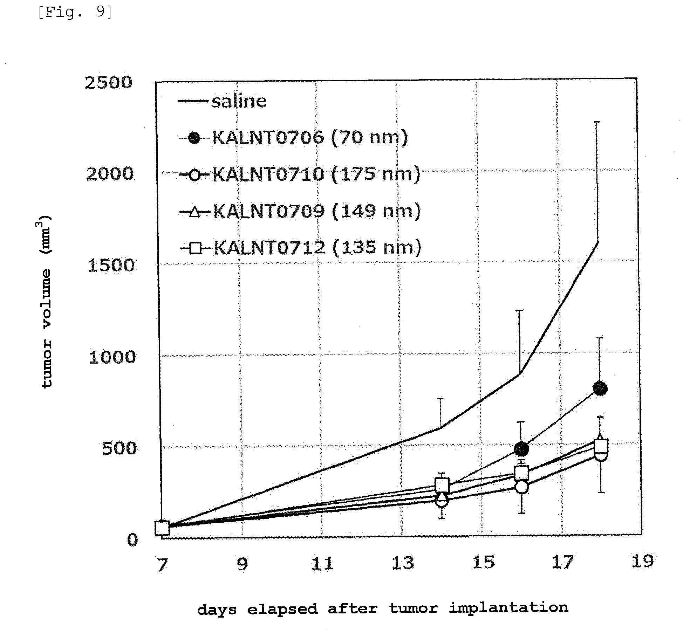

   Fig. 9 shows an in vivo evaluation of the pharmacological activity of K3-LNT. 2.5×10⁵ B16 tumor cells were subcutaneously implanted into C57BL/6 mice (the day of implantation is day 0), and K3-LNT was intratumorally administered 3 times in total (7, 9, and 11 days later). For comparison, physiological saline was administered. The horizontal axis indicates the number of days after subcutaneous implantation of B16, and the vertical axis indicates the tumor size of the K3-LNT and physiological saline administration groups.
   The lot numbers of the test substances used in Fig. 9 are as follows:
   ·KALNT0706:20201106-07
   ·KALNT0710:20201106-04
   ·KALNT0709:20201106-03
   ·KALNT0712:20201106-06.
[Fig. 10]

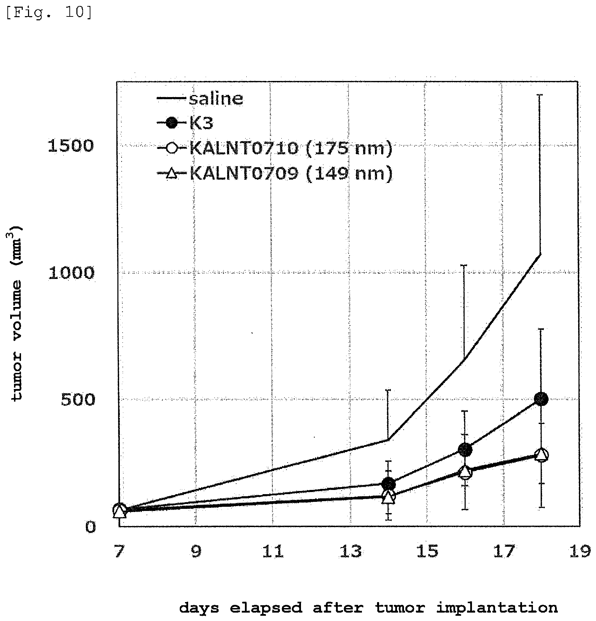

   Fig. 10 shows an in vivo evaluation of the pharmacological activity of K3-LNT, as in Fig. 9. The number of transplanted tumor cells was set to 5×10⁵. For comparison, physiological saline and K3 were administered.
   The lot numbers of the test substances used in Fig. 10 are as follows:
   ·KALNT0710:20201106-04
   • KALNT0709:20201106-03.

### [Description of Embodiments]

### 1. Complex of β(1→3) glucan and nucleic acid with controlled particle size

The present invention provides a complex of β(1→3) glucan and a nucleic acid having a controlled particle size. The present inventors have conducted intensive studies and found that the anticancer activity of a complex of β(1→3) glucan and a nucleic acid is remarkably high when the average particle size of the complex is within the range of 20 nm to 180 nm, preferably 20 nm to 130 nm or 40 nm to 180 nm, more preferably 80 nm to 130 nm or about 130 nm to 180 nm, which resulted in the completion of the present invention. Therefore, BG-CpG with an average particle size controlled to fall within such ranges can be used as a therapeutic drug or prophylactic drug for cancer immunotherapy and the like.

The β(1→3) glucan used in the present invention is not particularly limited as long as it has the property of forming a complex with a nucleic acid, and β(1→3) glucans such as schizophyllan (SPG), lentinan (LNT), curdlan, scleroglucan, pachyman, grifolan, laminan, and the like can be mentioned. It has already been reported that they have the property of forming complexes with nucleic acids (WO 2004/100965 and JP-B-4850512, immunostimulants). SPG and LNT can be mentioned as particularly preferred β(1→3) glucans that can be used in the present invention.

The complex of β(1→3) glucan and a nucleic acid in the present invention is not limited thereto, and is preferably a complex formed by β(1→3) glucan (BG) and a nucleic acid containing CpG ODN (BG-CpG). Further preferably, the complex in the present invention is a complex (K3-SPG) of SPG and CpG ODN with 40 bases of dA linked to the 3'-end of Type K CpG ODN (K3) (K3-dA40),or a complex (K3-LNT) of LNT and CpG ODN with 40 bases of dA linked to the 3'-end of Type K CpG ODN (K3) (K3-dA40) .

As described in detail in the following 4., SPG can be obtained by selecting a Schizophyllum commune strain with high SPG-producing ability, culturing same under appropriate culture conditions, causing efficient secretion of SPG into the medium, and purifying the medium. While SPG can be used as it is, it is used after controlling to an appropriate molecular weight as necessary. The method for controlling the molecular weight of β-glucan is also described in detail in 2. below.

In the present invention, the "complex with a controlled particle size" means a complex produced using β-glucan with an appropriate molecular weight as a constituent component in order to achieve a desired range of the average particle size of the complex of β-glucan and nucleic acid. Here, the molecular weight of β-glucan may not be specified. Furthermore, in the present invention, the "strictly controlled" means controlling the average particle size of the complex by a range of generally 10 nm to 50 nm or narrower. Such strict control can clarify the optimum average particle size range of a complex of β-glucan and nucleic acid for various pharmaceutical applications. Furthermore, such strict control makes it possible to produce a complex of β-glucan and nucleic acid having an average particle size that exerts the maximum pharmacological effect.

In WO 2016/098832, a series of SPGs with different molecular weights within the range of generally 10,000 to 5,400,000 were used to produce a complex with a nucleic acid called d(A)40-D35, and the relationship between the inertia radius and physiological activity is disclosed. According thereto, the control range of the inertia radius is 5 nm to 8 nm, 8 nm to 10 nm, 20 nm to 70 nm, 60 nm to 100 nm, 100 nm to 150 nm, or 150 to 200 nm, and the control width at the inertia radius of 20 nm or more is 50 nm or more. When this is converted to particle size, the control width thereof is generally 100 nm or more, and a strict control cannot be said to be performed. In addition, the strength of the pharmacological effect of these complexes is evaluated only semi-qualitatively in three stages, and no difference in the pharmacological effect is observed within the inertia radius range of 8 nm to 150 nm.

On the other hand, in the above-mentioned prior art (Miyamoto N., et al., Bioconjugate Chemistry 2017, 28, 565-573), attempts have been made to increase the particle size of a complex of β-glucan and nucleic acid containing SPG and CpG-dA40 as constituent components (CpG-SPG). Here, a nucleic acid (cCpG-dA40) having a sequence complementary to CpG was prepared, and a complex thereof with SPG was produced (cCpG-SPG). Then, by mixing CpG-SPG and cCpG-SPG, the CpG and cCpG of both complexes were bound to each other through noncovalent bonds, thus crosslinking the both complexes and forming a structure with a large particle size (CL-CpG nanogel). The inertia radius of CpG-SPG and cCpG-SPG was generally 10 nm, whereas the inertia radius of CL-CpG nanogel was generally 150 nm. The inertia radius of CL-CpG nanogel corresponds to an average particle size of generally 300 nm, and it was confirmed that CL-CpG nanogel has a stronger adjuvant effect than CpG-SPG.

As described above, even though the CL-CpG nanogel of the prior art successfully increased the particle size as compared with CpG-SPG, it cannot be said that the particle size could be strictly controlled. That is, the average particle size of around 300 nm is not strictly controlled, and it cannot be said that the average particle size of the complex of β-glucan and nucleic acid has been optimized.

As described above, there are examples in the prior art that have succeeded in increasing the average particle size to generally 300 nm. However, strict control, or controlling the average particle size of the complex by a range of generally 10 nm to 50 nm or narrower, has not been achieved. The inventors considered that such poorly strict particle size control may have prevented the complex of β-glucan and nucleic acid from exhibiting the remarkable pharmacological effect it originally has. That is, they considered it important to specify, by strict control of particle size, the average particle size that exerts the maximum pharmacological effect, and to develop a technique that enables the production of a complex of β-glucan and nucleic acid having such average particle size.

The molecular weight of β-glucan to be produced and contained may vary depending on the strain and variety. Therefore, it is possible to produce β-glucan with a large molecular weight by using the optimum strain type and culture/growth conditions. Moreover, since β-glucan, which is a natural polysaccharide, has a wide molecular weight distribution, even if the average molecular weight is small, it contains large molecular weight components. By fractionating and purifying same, β-glucan with a large molecular weight can be obtained. Furthermore, it is also known that the molecular weight can be increased by chemically or physically crosslinking polysaccharides having a given molecular weight (Basu A., et al., Bioconjugate Chemistry 2015, 26, 1396-1412; Pawar H. A., et al., Biology and Medicine (Aligarh) 2015, 6, 224) .

With these methods, the molecular weight of polysaccharides can be increased to infinity, that is, until it becomes insoluble as a gel. That is, by appropriately applying these methods, the molecular weight of β-glucan can be increased to infinity. Furthermore, it is also possible to reduce the molecular weight after once obtaining and producing high-molecular-weight β-glucan under the molecular weight control conditions and the like disclosed in the present invention. As already described, the prior art (WO2016/098832 and Miyamoto N., et al., Bioconjugate Chemistry 2017, 28, 565-573) shows the usefulness of a complex of β-glucan with an inertia radius of 150 nm and a nucleic acid. It is shown that a complex of β-glucan and nucleic acid having an average particle size of about 300 nm is medicinally useful by preparing high-molecular-weight β-glucan by any of the above-mentioned methods. However, in the prior art, the particle size is not strictly controlled, in other words, the average particle size is not controlled within a range of generally 10 nm to 50 nm or narrower, and therefor, an average particle size of 300 nm cannot be said to be optimal.

BG-CpG composed of SPG thus obtained and a nucleic acid (preferably K3-dA40, whose usefulness as a cancer immunotherapeutic agent has been confirmed), and having a strictly controlled particle size can be produced. Surprisingly, the present inventors found that the pharmacological effect of BG-CpG with an average particle size in the range of 80 nm to 130 nm or 130 nm to 180 nm is remarkably high in a pharmacological evaluation system assuming cancer immunotherapy. As already mentioned, the average particle size of BG-CpG, which has been widely used in conventional research, is around 30 nm, or 20 nm to 50 nm, and the average particle size found by the present inventors is outside such range.

In the Examples of the present specification, the above-mentioned BG-CpG with a strictly controlled particle size was evaluated for the effect as a cancer immunotherapeutic agent, by using the latest in vitro and in vivo pharmacological evaluation systems with due consideration of the suitability for clinical use. As a result, the present inventors found that the particle size and the pharmacological activity of BG-CpG are strongly related to each other and that BG-CpG with an average particle size in the range of 80 nm to 130 nm or 130 nm to 180 nm has a remarkably high pharmacological activity.

As shown in the following Examples, an in vitro evaluation system that uses hPBMCs to evaluate the IFN-α induction potency of BG-CpG, and an in vitro evaluation system that uses mouse spleen cells to evaluate the IFN-γ induction potency of BG-CpG were used to determine the optimal particle size of BG-CpG for pharmacological activity (anticancer activity). These in vitro evaluation systems are considered to have higher data stability and higher quantitativeness than mouse cancer transplantation models, which have high inter-experimental variation. In addition, the IFN-α induction level of BG-CpG showed a maximal effect (Emax) (what is called bell shape) at the middle dose. In order to improve quantitativeness, the IFN-α induction potency of BG-CpG was calculated from the area under the curve (hereinafter referred to as AUC) in the following Examples. Similarly, for IFN-γ, the amount of BG-CpG added was set to two levels of 2 µg/mL and 10 µg/mL, the IFN-γ production amount was measured after 24 hours of culture, and the total value of these was used as an index of the strength of pharmacological activity. As a result, BG-CpG exhibited a stronger pharmacological activity as the average particle size increased in the range of 20 nm to 130 nm or 40 nm to 180 nm. The average particle size of BG-CpG that has the maximum pharmacological activity was discovered for the first time by the strict control of the particle size in the present invention.

Furthermore, in the following Examples, the anticancer activity of BG-CpG, which was narrowed down by in vitro evaluation, was evaluated using a mouse model in which B16 melanoma cancer was transplanted (Kitahata, Y., et al. Circulating nano-particulate TLR9 agonist scouts out tumor microenvironment to release immunogenic dead tumor cells. Oncotarget 7, 48860-48869 (2016)). As a result, anticancer activity of BG-CpG was observed in vivo. As described above, it was found that the BG-CpG of the present invention with a strictly controlled particle size affords high anticancer activity.

### 2. Pharmaceutical composition containing β(1→3) glucan-nucleic acid complex with controlled particle size

The BG-CpG of the present invention can be used as a therapeutic agent or prophylactic drug/vaccine for various diseases, based on the activity mechanism thereof. The present invention provides a pharmaceutical composition containing the above-mentioned complex of the present invention. Prophylactic or therapeutic application for specific diseases is determined, the effectiveness and safety thereof are predicted or verified through exploratory studies, non-clinical tests, and clinical tests, and then the pharmaceutical composition is put to practical use. At some stage in the pharmaceutical product development process, the particle size of the BG-CpG of the present invention is optimized or its acceptance range is defined. Generally, in the exploratory study stage, the particle size is optimized or its acceptance range is determined using in vitro or in vivo activity thereof as an index. In addition, in safety tests in non-clinical tests, the appropriateness of the optimized particle size or the particle size range set as the acceptance range is validated from the safety aspect.

The pharmaceutical composition of the present invention can be obtained by formulating the above-mentioned complex of the present invention according to a conventional means. The pharmaceutical composition of the present invention contains the complex of the present invention and a pharmacologically acceptable carrier. Also, the pharmaceutical composition may further contain an antigen. Such pharmaceutical composition is provided in a dosage form suitable for oral or parenteral administration.

As a composition for parenteral administration, for example, injection, suppository and the like are used, and injection may encompass dosage forms such as intravenous injection, subcutaneous injection, intradermal injection, muscular injection, drip injection and the like. Such injections can be prepared according to known methods. The preparation methods of injection include dissolving or suspending the above-mentioned complex of the present invention in an aseptic aqueous solvent generally used for injection. As the aqueous solvent for injection, for example, distilled water; physiological saline; buffers such as phosphate buffer, carbonate buffer, tris buffer, acetate buffer and the like; and the like can be used. The pH of such aqueous solvent is, for example, 5 - 10, preferably 6 - 8. Prepared injection is preferably filled in a suitable ampoule.

It is also possible to prepare a powder preparation of the complex of the present invention by subjecting a suspension of the complex of the present invention to a treatment such as vacuum drying, freeze-drying and the like. The complex of the present invention can be used by preserving same in a powder state, and dispersing the powder in an aqueous solvent for injection when in use.

The content of the complex of the present invention in a pharmaceutical composition is generally about 0.1 - 100 wt%, preferably about 1 - 99 wt%, more preferably about 10 - 90 wt%, of the whole pharmaceutical composition.

The pharmaceutical composition of the present invention may contain, as an active ingredient, the complex of the present invention alone or a combination of the complex of the present invention and other active ingredient.

### 3. Pharmaceutical use

Since the complex of the present invention has a superior immunostimulating activity, the complex and pharmaceutical composition of the present invention can be used as immunostimulating agents. Administration of the complex or pharmaceutical composition of the present invention to a mammal (primates such as human and the like, rodents such as mouse and the like, etc.) can induce an immune reaction in the mammal. Particularly, since the complex of the present invention has the activating property as D type CpG ODN, and stimulates peripheral blood mononuclear cells to produce large amounts of type I interferon (Pan-IFN-α, IFN-α2 etc.) and type II interferon (IFN-y), it is useful as a type I interferon production inducing agent, type II interferon production inducing agent, or type I and type II interferon production inducing agent. Since the complex of the present invention and a pharmaceutical composition containing same induce production of both type I and type II interferons, they are useful for the prophylaxis or treatment of a disease for which either or both of type I and type II interferons is/are effective. Examples of the disease for which type I interferon is effective include viral infectious disease (e.g., hepatitis C virus (HCV), herpes virus, papilloma virus, RS virus, influenza virus etc.), cancer and the like. Examples of the disease for which type II interferon is effective include allergic disease, infectious diseases with intracellular parasitic protozoan (Leishmania etc.), bacterium (Listeria, Mycobacterium tuberculosis etc.) and the like, and the like. As for acute viral infectious diseases with RS virus, influenza virus and the like, since both type I interferon and type II interferon enhance immune responses relating to the virus elimination, the complex and a pharmaceutical composition containing same of the present invention are expected to be effective for acute viral infectious diseases.

Tumors that can be treated by the complex of the present invention and a pharmaceutical composition containing same are not particularly limited. The tumors are preferably exemplified by pancreatic cancer, liver cancer, gastric cancer, colorectal cancer, kidney cancer, breast cancer, uterine cancer (e.g., cervical cancer), ovarian cancer, lung cancer, thyroid cancer, skin cancer (e.g., melanosarcoma), head and neck cancer, sarcoma, prostate cancer, bladder cancer, brain tumor, gastrointestinal stromal tumor(GIST), leukemia (e.g., acute myeloid leukemia (AML), chronic myeloid leukemia (CML), or chronic lymphocytic leukemia (CLL) or acute lymphocytic leukemia (ALL)), lymphoma or malignant lymphoma (e.g., T lymphoma (including adult T cell leukemia), B cell lymphoma, non-Hodgkin lymphoma or diffuse large B cell lymphoma (diffuse large B-cell lymphoma, DLBCL)), more preferably melanoma, T lymphoma, colorectal cancer, pancreatic cancer, chronic myelogenous leukemia, liver cancer, cervical cancer, ovarian cancer, bladder cancer, adult T cell leukemia, further preferably melanoma, T lymphoma (including adult T cell leukemia), colorectal cancer, and pancreatic cancer, that are cancers highly sensitive to IFN-α and IFN-γ, which are cytokines strongly induced by the complex of the present invention and a pharmaceutical composition containing same (Non Patent Literature 26 and Martin-Hijaro, L. and Sainz Jr., B. 2020: The interactions between cancer stem cells and the innate interferon signaling pathway. Frontiers in immunology 11, Article 526.).

In addition, the complex of the present invention has a strong vaccine adjuvant activity, and administration of the complex of the present invention together with an antigen to a mammal can induce a strong immune reaction to the antigen. Therefore, the present invention also provides a composition for inducing an immune reaction to an antigen, which contains (a) the complex of the present invention, and (b) the antigen. The complex of the present invention strongly induces both a humoral immune reaction to an antigen (antigen-specific antibody production) and a cellular immune reaction (antigen-specific CTL induction). .Therefore, the complex of the present invention and a pharmaceutical composition containing same are useful as vaccine adjuvants.

In the present specification, the adjuvant refers to a pharmaceutical aid that promotes immune responses, which is a substance that non-specifically enhances immune responses to an antigen when administered with the antigen to the living body.

The antigen is not particularly limited as long as it has antigenicity for a mammal (primates such as human and the like, rodents such as mouse and the like, etc.) to be the subject of administration, and can be recognized as an antigen by an antibody or cytotoxic T lymphocyte (CTL, CD8⁺ T cells). Any substance that becomes an antigen (protein, peptide, nucleic acid, lipid, carbohydrates, and a modification of the aforementioned substance (e.g., modification introduced with deletion, substitution, and/or addition of one or more amino acids (hereinafter mutation etc.) and the like) can be used. As the antigen, antigen derived from pathogen such as protozoa, fungi, bacterium, virus and the like, and an antigen relating to cancer or a particular disease can also be used.

In the present specification, "antigen A derived from a pathogen X" means that antigen A is contained as a constituent factor in the pathogen X. For example, when antigen A is a polypeptide, it means that the amino acid sequence of the polypeptide is present in the amino acid sequence of the protein encoded by the genome of pathogen X.

Examples of the antigen derived from a pathogen include the pathogen itself or a part thereof, an inactivated or attenuated pathogen itself or a part thereof, or a modification introduced with a mutation thereto and the like, and the like.

When an antigen derived from a pathogen is used as the antigen, an immune reaction to the antigen is induced, and a mechanism for immunologically eliminating the pathogen containing the antigen from the body is constructed. Therefore, a composition comprising (a) the complex of the present invention, and (b) an antigen derived from a pathogen, for inducing an immune reaction to the antigen, is useful for the prophylaxis or treatment of the pathogen.

The complex of the present invention strongly induces both a humoral immune reaction (antigen-specific antibody production), and a cellular immune reaction (antigen-specific CTL induction) to the antigen. Therefore, an antigen derived from an intracellular infectious pathogen (virus, protozoa, fungi, bacterium etc.) known to be recognized by cytotoxic T lymphocytes, an antigen related to cancer cells (e.g., tumor antigen) and the like are preferably used as the antigen.

While the intracellular infectious virus is not particularly limited, examples thereof include RS virus, influenza virus, parainfluenza virus, hepatitis C virus (HCV), hepatitis A virus (HAV), hepatitis B virus (HBV), Ebolavirus, cytomegalovirus, adenovirus, polio virus, Japanese encephalitis virus, measlesvirus, mumpsvirus, rubellavirus, rabiesvirus, yellow fever virus, varicellaherpes zostervirus, hantavirus, dengue virus, norovirus, rotavirus, parvovirus, corona virus, distemper virus, adult T cell leukemia virus (HTLV-1), human immunodeficiencyvirus (HIV), herpes virus, papilloma virus and the like. Examples of the intracellular infectious bacteria include mycoplasma and the like. Examples of the intracellular infectious protozoa include plasmodium, schistosoma and the like. The intracellular infectious pathogen is preferably virus (specifically, RS virus, or influenza virus etc.).

Examples of the antigen related to cancer cells include protein, sugar chain, peptide that are specifically expressed by cancer cells, variant of the aforementioned substances (deleted, substituted, and/or added) or modification thereof and the like.

Since the complex of the present invention strongly induces both the type I and type II interferons, in one embodiment, a virus (e.g., RS virus, influenza virus) that causes acute viral infectious disease, for which both the type I interferon and type II interferon are effective, is selected as the virus.

For example, a composition containing (a) the complex of the present invention, and (b) an antigen derived from a pathogen or cancer, for inducing an immune reaction to the antigen, is administered to a patient with the pathogen infection or cancer or a human potentially affected with the pathogen infection or cancer, to antigen-specifically activate cytotoxic T lymphocytes (CTLs) in the subject who received the administration, induce the antigen-specific antibody production, i.e., induce a protective immune reaction in the warm-blooded animal (preferably, human), whereby the infection and cancer can be prevented or treated. Accordingly, the composition is useful as a vaccine for the prophylaxis or treatment of the above-mentioned diseases such as infection, cancer and the like.

In addition, since the complex of the present invention can strongly induce both a humoral immune reaction (antigen-specific antibody production) and a cellular immune reaction (antigen-specific CTL induction) to an antigen, any of a surface antigen and an internal antigen of pathogen and cancer cell can be used as the antigen, and use of a mixture of a surface antigen and an internal antigen is also desirable.

A composition comprising (a) the complex of the present invention, and (b) an antigen, for inducing an immune reaction to the antigen, can be prepared according to the above-mentioned pharmaceutical composition of the present invention.

The complex of the present invention or the pharmaceutical composition of the present invention can be administered concurrently with or separately from other medicaments. For example, the complex of the present invention or the pharmaceutical composition of the present invention may be administered after other medicaments are administered, or other medicaments may be administered after the complex or the pharmaceutical composition is administered, or the complex or the pharmaceutical composition and other medicaments may be administered simultaneously. Examples of other medicaments include various anticancer agents such as chemotherapeutic agents, radiation therapy and the like, and a pharmaceutical composition containing further medicaments in addition to the complex or pharmaceutical composition of the present invention is also encompassed in the present invention.

TLR-9 agonists exert remarkable antitumor effects when used in combination with immune checkpoint inhibitors (Adamus, T. and Kortylewski, M., The revival of CpG pligonucleotide-based cancer immunotherapies, Contenp. Oncol. (Pozn.) 21 (1A), 56-60 (2017).). Immune checkpoint inhibitors include PD1 inhibitors, PD-L1 inhibitors, and CTLA-4 inhibitors, and anti-PD-1 antibodies, anti-PD-L1 antibodies, and anti-CTLA-4 antibodies are respectively known (Bagchi, S., Yuan, R., and Engleman, E.G., Immune checkpoint inhibitors for the treatment of cancer: clinical impact and mechanism of response and resistance, Ann. Rev. Pathol. 16, 223-249 (2021).). The effect of the combined use of a TLR-9 agonist called IMO-2125 and an anti-PD-1 antibody has been verified in an animal model of pancreatic cancer (Carbone C., et al., Intratumoral injection of TLR-9 agonist promotes an immunopermissive microenvironment transition and causes cooperative antitumor activity in combination with anti-PD1 in pancreatic cancer, J. Immunother. Cancer 9 2021). Therefore, the combined use of the complex of the present invention or the pharmaceutical composition of the present invention as a TLR-9 agonist and an immune checkpoint inhibitor may provide a useful therapeutic method.

### 4. Production method of β(1→3) glucan-nucleic acid complex with average particle size in specific range

The present invention also provides a method for producing a complex of β(1→3) glucan and a nucleic acid, having an average particle size within a specific range. In the present invention, BG-CpG with a strictly controlled particle size was successfully produced using starting materials with known sources of supply and by a feasible production method.

As already mentioned, SPG is a β(1→3) glucan produced by a strain of Schizophyllum commune, and a plurality of the strain are known. The present inventors have intensively studied the amounts of SPG produced by different strains of Schizophyllum commune, and have found that the productivity of a specific strain is particularly high. Therefore, it is a preferred embodiment of the present invention to obtain SPG by using a Schizophyllum commune strain with high SPG productivity. Similarly, lentinan (LNT) is a β(1→3) glucan contained in the shiitake mushroom fruiting body, and extraction of LNT from the shiitake mushroom fruiting body is a preferred embodiment of the present invention.

### (1) Production method of β-glucan using Schizophyllum commune strain

In order to produce β-glucan using Schizophyllum commune strain, etc., the Schizophyllum commune strain is cultured in a medium in which the strain can grow, and SPG is produced in a medium outside the fungal cells, according to the methods well known to those skilled in the art (Shoichi KIKUMOTO, et al., Polysaccharide Produced by Schizophyllum commune, Nippon Nogeikagaku Kaishi, 44, 337-342 (1970); Takashi MIZUNO and Masamitsu KAWAI, ed., Kinoko no Kagaku/Seikagaku, published by Gakkai Shuppan Center(1992)). As the medium, a normal medium containing nutrient sources (carbon source, nitrogen source, inorganic salts) normally available to strains belonging to Schizophyllum commune and, where necessary, organic nutrient sources can be used. A culture medium containing saccharides as a carbon source is preferred. As these media, any of various synthetic media, semi-synthetic media, natural media, and the like can be used.

As the above-mentioned carbon source, saccharides are preferred, and the saccharides include monosaccharides such as glucose, fructose, mannose, galactose, xylose, and arabinose, disaccharides such as sucrose, maltose, lactose, and trehalose, oligosaccharides such as fructo-oligosaccharide and xylooligosaccharide, and polysaccharides such as dextrin and starch. These can be used alone or in combination. Among these, hexoses such as glucose and fructose, disaccharides such as sucrose and lactose, starch and dextrin, and polysaccharides such as hydrolysates of these carbohydrates are preferably used as the main carbon sources. Also, beet pomace, sugarcane pomace, fruit pomace of citrus and the like, or pomace obtained by adding sugar to these pomaces, and the like can also be used. In addition, alcohols such as glycerol and ethylene glycol, sugar alcohols such as mannitol, sorbitol, and erythritol, and other carbon sources such as organic acids can be used as appropriate. These carbon sources may be added at any time during the culture. For example, by appropriately feeding saccharides such as glucose into the medium, the production rate and production amount of β-glucan can be relatively increased.

As the above-mentioned nitrogen source, organic nitrogen sources such as peptone, meat extract, soy flour, casein, amino acid, malt extract, corn steep liquor, casein decomposition product, yeast extract, and urea, and inorganic nitrogen sources such as sodium nitrate, ammonium sulfate, ammonia gas, and aqueous ammonia can be used alone or in combination.

As the above-mentioned inorganic salts, for example, salts such as sodium chloride, potassium chloride, calcium carbonate, magnesium sulfate, sodium phosphate, potassium phosphate, and cobalt chloride, heavy metal salts, and the like can be used. Where necessary, vitamins can also be added and used. When foaming occurs during culture, various known antifoaming agents can also be added as appropriate to the medium.

The conditions for culturing the strain of the present invention are not particularly limited, and can be appropriately selected within a range in which the strain can grow well. Generally, it is preferable to culture at pH 5.0 to pH 8.5 and 20°C to 35°C for about 5 to 14 days. These culture conditions can be appropriately changed according to the type and properties of the strain to be used, external conditions, and the like and the optimal conditions can be selected. The amount of strain to be inoculated into the medium is preferably 1% or a platinum loop for flask culture, and a seed culture medium in 1%(v/v) to 10%(v/v) of the culture medium is preferably added for scale-up. The addition is not limited to these as long as the culture is substantially possible.

Culture of the strain of the present invention is performed under aerobic conditions such as aeration stirring and shaking. Culture is preferably performed until the production concentration of the target β-glucan is reached, which is generally for 5 to 14 days. Alternatively, β-glucan may be continuously produced by continuously adding saccharides and medium components that are substrates for β-glucan.

After culturing as described above, β-glucan produced by secretion outside the fungal cells is separated and collected from the culture medium according to a conventional method. Specifically, various known purification means may be selected and combined, such as separating and removing solids such as fungal cells from the culture medium by centrifugation, filtration, and the like, removing impurities and salts with activated carbon, ion exchange resin, and the like. Furthermore, for example, separation and purification can be performed by using adsorption and elution to hydrophobic resin, solvent precipitation using ethanol, methanol, ethyl acetate, n-butanol, etc., column method using silica gel, etc. or thin layer chromatography, preparative high performance liquid chromatography using reversed phase column, and the like, alone or in appropriate combinations, or repeatedly depending on the case.

In addition, the fungal cells may be sterilized before and after separating, by the above-mentioned method, the β-glucan produced by secretion outside the fungal cells. The sterilization temperature is not particularly limited as long as it is a temperature at which the fungal cells are killed, and is 90°C or more, or 121°C under pressure. Any time can be set as the sterilization time, and 15 to 60 minutes is preferred.

### (2) Production method of β-glucan using shiitake mushroom fruiting body

Lentinan is a β-glucan contained in the shiitake mushroom fruiting body. Therefore, lentinan can be obtained by extracting and purifying from shiitake mushroom fruiting body. In the production of the lentinan of the present invention, shiitake mushroom fruiting body is cultivated by a method such as log cultivation or mushroom bed cultivation, and lentinan can be extracted from the fruiting body, according to methods well known to those skilled in the art (Kiyotoshi Omori, Hiroshi Koide, All About Mushroom Cultivation, published by Rural Culture Association Japan (2001); Editorial Committee of All About Current Biotechnology, ed., Cultivation and Breeding of Mushrooms, published by Nogyo-Tosho (1992)), or a novel method.

Log cultivation is a method for cultivating mushrooms of wood-rotting fungi, mainly using sawthorn oak, Quercus serrata, and Quercus crispula, which involves planting fungi on logs that have been cut and dried. Innoculation of fungi and growth control are conducted and the developed shiitake mushrooms are harvested.

In mushroom bed cultivation, one or more kinds of pulverized wood materials such as sawdust, chip dust, and chips of broad-leaf trees are mixed with one or more kinds of ground products of grains such as bran and rice bran, and beans, water is added to adjust the water content of the medium, and the medium is pressurized and bagged, sterilized, and cooled in accordance with a conventional method to prepare a mushroom bed. Shiitake mushroom inoculum is inoculated, and the mushroom bed is managed for culture for a certain period of time and subjected to normal cultivation (cultivation method in which the mushroom bed is taken out from the bag and mushrooms are grown from the entire medium) or top culture (only the upper part of the mushroom bed is exposed and mushrooms are cultivated only from the upper part of the medium), and the developed shiitake mushrooms are harvested.

Extraction and purification of lentinan can be performed according to methods well known to those skilled in the art (Chihara et al. Fractionation and purification of the polysaccharides with marked antitumor activity, especially lentinan, from Lentinus edodes (Berk.) Sing. Cancer Research 30, 2776-2781 (1970); Goro Chihara, Cancer and Enhanced Immunity, Kodansha Ltd. (1980)). Alternatively, extraction and purification of lentinan can also be performed by a novel method not publicly known.

The β-glucan contained in the shiitake mushroom fruiting body is produced from the fruiting body according to a conventional method. Specifically, various known purification means can be selected and combined, such as extracting the cut fruiting body with hot water and removing impurities and salts with activated carbon, ion exchange resin, or the like. Furthermore, for example, separation and purification can be performed by using solvent sedimentation using ethanol, methanol, ethyl acetate, n-butanol, etc., adsorption to and elution from hydrophobic resin, column method using silica gel, etc. or thin layer chromatography, preparative high performance liquid chromatography using reversed phase column, and the like, alone or in appropriate combinations, or repeatedly depending on the case.

In the middle of these production steps or in the final step, washing with an organic solvent or aqueous medium is performed as necessary, and freeze drying, drying under reduced pressure, and the like are performed by methods well known to those skilled in the art to obtain the desired product.

### (3) Method for controlling molecular weight of β-glucan

Examples of methods for controlling the molecular weight of β-glucan include, but are not limited to, enzymatic decomposition, alkali hydrolysis, acid hydrolysis, shearing force by a high-pressure homogenizer, and decomposition by ultrasonic energy. An appropriate method can be selected from these and used appropriately (Toshio Miyazaki, ed., Structure and Physiological Activity of Polysaccharides, Asakura Publishing Co., Ltd., (1990)).

### (4) Method for determining β-glucan structure

The structure of the obtained β-glucan can be determined by a method selected from or a combination of methods such as ¹H-NMR, ¹³C-NMR, infrared absorption spectrum, elemental analysis, and the like, like general organic compounds.

When using dimethyl sulfoxide-d6 (DMSO-d6), which is a measurement solvent widely used for the measurement of the nuclear magnetic resonance spectrum (hereinafter to be referred to as NMR) of β-glucan, signals of hydroxyl groups are also observed in ¹H-NMR and give a very broad spectrum. On the other hand, 0.25 M sodium deuteroxide/heavy water dissolves β-glucan comparatively easily than DMSO-d6. In ¹H NMR, since signals of hydroxyl groups are not observed, the characteristics of the glucan-constituting unit can be clearly seen. When 0.25 M sodium deuteroxide/heavy water is used as the measurement solvent, β-glucan tends to decompose at room temperature. Thus, a spectrum suitable for the analysis can be obtained by optimizing the NMR spectrum measurement temperature conditions and the necessary amounts of samples.

The molecular weight and molecule size can be specified as absolute numerical values or as relative numerical values corresponding to standard substances with known molecular weights, or as scales with a certain degree .of correlation, by methods well known to those skilled in the art such as multi-angle light scattering method, size exclusion chromatography (SEC), dynamic light scattering, and the like. β(1→3) glucan (BG) is a chain molecule, and exists in a state of three chains entangled in a neutral aqueous solution, but exists in a state of being dissociated into single chains in an alkaline solution (WO 2015/041318, COMPLEX CONTAINING OLIGONUCLEOTIDE HAVING IMMUNOPOTENTIATING ACTIVITY AND USE THEREOF; Japanese Patent No. 5605793, Method for preparing complex of β-1,3-glucan/nucleic acid (Japanese Patent Application No. 2010-043592); Daijiro TANOHATA, Yusuke SANADA, Shinichi MOCHIZUKI, Noriko MIYAMOTO, and Kazuo SAKURAI, Dilute Solution Properties of Polysaccharide/Nucleic Acid Complexes, Kobunshi Ronbunshu, 72, 37-47, (2015)). Relative molecular weight can be estimated by size-exclusion chromatography method (SEC method) under alkaline conditions using pullulan, which has a chain structure like BG, as a molecular weight reference standard. Furthermore, the absolute molecular weight and molecular size can be estimated by the static light scattering method, concurrently using a multi-angle light scattering detector (MALLS) as a chromatography detector (Wyatt, P. J. Anal Chim Acta 1993, 272, 1; Podzimek, S. J Appl Polym Sci 1994, 54, 91).

### (5) Production method of nucleic acid

For nucleic acids, K3-dA40 and the like can be produced based on synthesis techniques and nucleic acid chemistry well known to those skilled in the art. Such production methods are described, for example, in (1) Gait, M.J., (1985). Oligonucleotide Synthesis: A Practical Approach, IRL Press, (2) Gait, M.J. (1990). Oligonucleotide Synthesis: A Practical Approach, IRL Press, (3) Eckstein, F. (1991). Oligonucleotides and Analogues:A Practical Approach, IRL Press, (4) Adams,R.L. et al. (1992). The Biochemistry of the Nucleic Acids, Chapman& Hall, (5) Shabarova, Z. et al. (1994). Advanced Organic Chemistry of Nucleic Acids, Weinheim; Blackburn, G.M. et al. (1996). Nucleic Acids in Chemistry and Biology, Oxford University Press, (6) Hermanson, G.T. (1996). Bioconjugate Techniques, Academic Press, and the relevant portions of these documents are incorporated herein by reference. Furthermore, the method described in Stein et al., 1988, Nucl. Acids Res. 16:3209 can also be used to synthesize phosphorothioate-oligonucleotide. Furthermore, the method described in Sarin et al., 1988, Proc. Natl. Acad. Sci. USA 85:7448-7451 can also be used to prepare methylphosphonate-oligonucleotide by using controlled pore glass polymer support and the like.

### (6) Production method of BG-CpG

Using the β-glucan and nucleic acid obtained as described above as starting materials, BG-CpG can be produced according to methods well known to those skilled in the art ((1) WO 2016/103531 and PCT/JP2014/084772, USE OF NUCLEIC ACID-POLYSACCHARIDE COMPLEXES HAVING IMMUNOPOTENTIATING ACTIVITY AS ANTI-TUMOR DRUG, (2) WO 2015/041318, COMPLEX CONTAINING OLIGONUCLEOTIDE HAVING IMMUNOPOTENTIATING ACTIVITY AND USE THEREOF, (3). Japanese Patent No. 5605793, Method for preparing a complex of β-1,3-glucan/nucleic acid (Japanese Patent Application No. 2010-043592)).

That is, considering that the stoichiometric ratio of the number of sugar units constituting the main chain of β-glucan (hereinafter as mG) and the number of bases of nucleic acid (when deoxyadenosine is used as the nucleic acid that interacts with β-glucan, hereinafter dA) is 2:1, that is, the mG/dA ratio is 2, the mixing ratio of one component is increased or decreased as appropriate according to the purpose. Since β-glucan generally forms a helical structure or an aggregate, it is subjected before complexing to an alkali treatment or dissolved in an aqueous medium containing N,N-dimethylsulfoxide to form a monomer, and then used to form a complex. Alternatively, both components are allowed to coexist in these media, and a complex is formed by neutralizing the system or diluting or removing the organic solvent. Property analysis of BG-CpG is performed in order to confirm or ensure its structural characteristics and general quality property that a pharmaceutical product should possess.

The BG and CpG oligonucleotide complex (BG-CpG) exists stably in a neutral aqueous solution and has good solubility, which makes it possible to measure the particle size by the dynamic light scattering method (DLS method). When a laser beam is irradiated on particles in a solution or suspension undergoing Brownian movement, the scattered light from the particles fluctuates according to the diffusion coefficient. Since large particles move slowly, the scattered light intensity fluctuates moderately, whereas small particles move quickly and thus the scattered light fluctuates rapidly. In the DLS method, fluctuations in scattered light reflecting this diffusion coefficient are detected, and the particle size is measured using the Stokes-Einstein equation. The average particle size and particle size distribution obtained by this method are one of the important factors that indicate the characteristics of mainly the colloidal dispersion preparations (The Japanese Pharmacopoeia 17th edition, General Information, "Measurement of the Diameter of Particles Dispersed in Liquid by Dynamic Light Scattering" P2346-2348). The DLS method includes the photon correlation method and the frequency analysis method depending on the difference in the analysis method of the detected signals, and is applied to particles with a particle size of several nm to about 1 µm or particles before the influence of sedimentation is observed. In the Examples of the present specification, the photon correlation method was used, and the average particle size based on the scattered light intensity was obtained by Cumulant analysis from the autocorrelation function of the scattered light intensity (The Japanese Pharmacopoeia 17th edition, General Information, "Measurement of the Diameter of Particles Dispersed in Liquid by Dynamic Light Scattering" P2346-2348; ISO 22412: 2008 Particle size analysis - Dynamic light scattering (DLS)). Here, when the particle size distribution of the test substance is unimodal, the particle size at the peak top is the average particle size. In the present invention, correlation was investigated between the average particle size and the pharmacological activity of BG-CpG. On the other hand, when the particle size distribution was bimodal or multimodal, the peak showing the maximum light scattering intensity was selected, and the correlation between the particle size of the peak top thereof, that is, the average particle size of the peak, and the pharmacological activity of BG-CpG was investigated.

The present invention is now explained in more detail in the following by referring to Examples, Experimental Examples, and pharmaceutical compositions, which are not to be construed as limitative.

### [Example]

### Example 1: Production of SPG by fungal cell culture

The Schizophyllum commune strain having high SPG-producing ability was selected from the 33 strains of Schizophyllum commune isolated from the samples collected and held in Japan by the present inventors. The Schizophyllum commune strain was sufficiently grown on a Potato Dextrose Agar (PDA) slant, and 1/4 of the fungal cells grown on the surface of the PDA slant were homogenized with 5 mL of sterilized water, and inoculated into 80 mL of an SPG medium sterilized in advance (glucose 3.0%, yeast extract 0.3%, ammonium dihydrogen phosphate 0.15%, potassium dihydrogen phosphate 0.1%, magnesium sulfate heptahydrate 0.05%, (pH 4.8 before sterilization)) and contained in one 500 mL Erlenmeyer flask. The culture was conducted by shaking the 500 mL Erlenmeyer flask inoculated with the fungal cells at 210 rpm, 28°C for 7 days (168 hr) using a rotary shaker.

After completion of the culture, 45 mL or 15 mL of the obtained culture medium was centrifuged (10000 rpm, 10 min) to separate the culture medium into culture supernatant and fungal cells. Methanol was added to 30 mL or 10 mL of the obtained culture supernatant to prepare a 35% methanol solution. A precipitate formed by the addition of methanol was centrifuged (10000 rpm, 10 min) and collected. The collected precipitate was washed with 10 mL or 5 mL of anhydrous methanol added thereto, and centrifugation was performed again. 1 mL to 3 mL of sterilized water was added to the precipitate, and the mixture was allowed to stand for about 1 hr and freeze-dried to obtain an SPG extract.

### Experimental Example 1: Evaluation of SPG productivity of each strain of Schizophyllum commune

The amount of the SPG extract obtained in Example 1 was weighed. The productivity of the SPG extract of each strain was evaluated using, as an index, the concentration of the SPG extract in the culture supernatant as calculated from the amount of the SPG extract and the amount of the culture supernatant.

As shown in Table 1, it was confirmed that the F-19065, F-30213, F-82046, F-33504, F-15977, and F-15537 strains have high SPG productivity. It was also confirmed that the F-19065, F-30213, F-82046, and F-33504 strains have very high SPG productivity.

**[Table 1]**

| Producibility of SPG extract in each strain | | |
|---|---|---|
| strain No. | species | SPG extract concentration µg/mL |
| **F-19065** | Schizophyllum commune | **1963** |
| **F-30213** | Schizophyllum commune | **1575** |
| | **F-82046** Schizophyllum commune | **1320** |
| **F-33504** | Schizophyllum commune | **1190** |
| **F-15917** | Schizophyllum commune | **920** |
| **F-15537** | Schizophyllum commune | **630** |
| **F-16516** | Schizophyllum commune | **440** |
| **F-16957** | Schizophyllum commune | **360** |
| **F-12187** | Schizophyllum commune | **320** |
| **F-15834** | Schizophyllum commune | **314** |
| **F-15513** | Schizophyllum commune | **145** |
| **F-15512** | Schizophyllum commune | **100** |
| **F-37343** | Schizophyllum commune | **100** |
| **F-33189** | Schizophyllum commune | **100** |
| **F-33262** | Schizophyllum commune | **60** |
| **F-30226** | Schizophyllum commune | **55** |
| **F-31452** | Schizophyllum commune | **50** |
| **F-30205** | Schizophyllum commune | **46** |
| **F-17081** | Schizophyllum commune | **40** |
| **F-80914** | Schizophyllum commune | **37** |
| **F-80270** | Schizophyllum commune | **32** |
| **F-17079** | Schizophyllum commune | **30** |
| **F-81418** | Schizophyllum commune | **25** |
| **F-18435** | Schizophyllum commune | **20** |
| **F⁻15010** | Schizophyllum commune | **14** |
| **F-19030** | Schizophyllum commune | **12** |
| **F-38177** | Schizophyllum commune | **11** |
| **F-82454** | Schizophyllum commune | **10** |
| **F-18434** | Schizophyllum commune | **10** |
| **F-12986** | Schizophyllum commune | **10** |
| **F-80255** | Schizophyllum commune | **10** |
| **F-356** | Schizophyllum commune | **10** |
| **F-81404** | Schizophyllum commune | **5** |

### Example 2: Production of SPG by fungal cell culture of SPG high-production strain

Two strains of Schizophyllum commune strain F-82046 and F-30213 having high SPG productivity were selected and used for the production of SPG.

In the same manner as in Example 1, 1/4 of the fungal cells grown on the surface of PDA slant were homogenized with 3 mL of sterilized water, and inoculated into 80 mL of an SPG medium sterilized in advance and contained in one 500 mL Erlenmeyer flask. Preculture was conducted by shaking the 500 mL Erlenmeyer flask inoculated with the fungal cells at 210 rpm and 28°C for 4 days using a rotary shaker. 5 mL of the preculture medium was homogenized and inoculated to a 500 mL Erlenmeyer flask containing 80 mL of SPG medium. Main culture was conducted by shaking the 500 mL Erlenmeyer flask inoculated with the preculture medium at 210 rpm and 28°C for 10 days using a rotary shaker.

After the completion of the culture, the whole culture medium was dispensed into centrifugal tubes and centrifuged (10,000 rpm, 10 min). Since the obtained supernatant had a high viscosity, a 1.5- to 1-fold amount of sterilized water was added, and suction filtration was performed to remove floating cells. Methanol was added to the filtrate to 35%, and the mixture was allowed to stand overnight after stirring. The deposited precipitate was centrifuged (10,000 rpm, 10 min) and washed with methanol. Then, the precipitate was infiltrated with sterilized water and freeze-dried. This freeze-dried product was used as the SPG extract.

The SPG extract was dissolved in a 1 M sodium hydroxide aqueous solution at a concentration of 1 mg/mL and gently shaken at 5°C for 24 hr. Thereafter, the mixture was filtered through an Omnipore membrane filter (manufactured by Merck Millipore) with a pore size of 5 µm and then the filter with a 1 µm pore size, and a 2-fold amount of methanol was added to the obtained filtrate to precipitate SPG. The precipitated SPG was washed several times with a mixed solution of methanol:water=3:1. This washing was repeated until the solution became neutral, and further washing once each with methanol and acetone was performed. Finally, the precipitate was dried at 40°C to obtain purified SPG before controlling the molecular weight (massive SPG, hereinafter mSPG).

The mSPG produced using Schizophyllum commune strain F-82046 was designated as mSPG-1a, and the SPG derived from Schizophyllum commune strain F-30213 was designated as mSPG-2a. In addition, using two lots of SPG purchased as reagents (manufacturer's lot number SCZ-37-01A is iSPG-1 in the present invention, and lot number SCZ-36-1 is iSPG-2 in the present invention) as standard samples, the ¹H-NMR and ¹³C-NMR spectra of these mSPGs were obtained and compared. Here, the solvent used was 0.25 M sodium deuteroxide/heavy water, and the optimum measurement temperature for simultaneously achieving a high S/N ratio and compound stability was found to be 15°C, and measurements were performed at this temperature. As shown in Fig. 1 and Fig. 2, the ¹H-NMR spectrum and ¹³C-NMR spectrum of mSPG-1a and mSPG-2a both matched the spectrum of the SPG standard sample, and no remarkable signals derived from impurities were observed. From the above, it was confirmed that the two lots of mSPG produced in this Example had high purity.

### Example 3: Production of SPG by fungal cell culture using large flask

In order to produce a large amount of SPG, culture was performed using an Ultra Yield Flask 2.5 L (manufactured by Thomson Instrument Company). As the Schizophyllum commune, F-82046 strain was used. Culture was performed in three stages: pre-preculture, pre-culture, and main culture. For fungi inoculation, one frozen seed of Schizophyllum commune strain F-82046 was used.

For the production of frozen seeds of Schizophyllum commune strain F-82046, 1/4 of the Schizophyllum commune fungal cells grown on the surface of PDA slant were homogenized with 3 mL of sterilized water, and inoculated into 80 mL of an SPG medium sterilized in advance and contained in one 500 mL Erlenmeyer flask. The culture was conducted by shaking the 500 mL Erlenmeyer flask inoculated with the fungal cells at 210 rpm and 28°C for 3 days using a rotary shaker. After 7 mL of glycerol sterilized in advance was added and blended with 63 mL of the culture medium, and the mixture was dispensed by 1.8 mL into Cryotube (manufactured by Thermo Fisher Scientific), frozen and stored in a freezer at -80°C.

For pre-preculture, one frozen seed was thawed at room temperature, and entirely inoculated into 80 mL of an SPG medium sterilized in advance and contained in one 500 mL Erlenmeyer flask. Preculture was conducted by shaking the 500 mL Erlenmeyer flask inoculated with the fungal cells at 210 rpm and 28°C for 3 days using a rotary shaker. For preculture, the pre-preculture medium (20 mL) was homogenized and entirely inoculated into 1000 mL of an SPG medium sterilized in advance and contained in an Ultra Yield Flask 2.5 L. Preculture was conducted by shaking the 2.5 L flask at 130 rpm and 28°C for 3 days using a rotary shaker. For the main culture, the preculture medium (10 mL) was inoculated into 1000 mL of an SPG medium sterilized in advance and contained in an Ultra Yield Flask 2.5 L. Main culture was conducted by shaking the 2.5 L flask at 130 rpm and 28°C for 11 days using a rotary shaker. SPG production amount was not fluctuated among multiple flasks, and the average concentration of SPG in the medium was about 2,000 µg/mL. After completion of the culture, distilled water was added so that the liquid volume in each flask was 1.5 L, and autoclave sterilization (121°C, 20 min) was performed. After the sterilization, the culture medium was further diluted with an equal volume of distilled water, the cells were removed with a nylon bag (pore size: 150 µm), and suction filtration was performed using filter paper (No. 2, manufactured by Advantec). Using a Smoothflow pump (Q series, Takumina Co., Ltd.), the treated filtrate obtained was mixed with methanol while adjusting the methanol concentration to 30% to 35%, and allowed to stand overnight to obtain a precipitate. A floating fraction consisting of this precipitate and a gel-like floating substance in the liquid were collected using tweezers, a ladle, and a nylon bag. The collected precipitate was centrifuged (8,000 rpm, 30 min) and washed with methanol. Then, the precipitate was infiltrated with sterilized water and freeze-dried to obtain an SPG extract as a freeze-dried product (SPG-247:yield 10.4 g).

A part (151 mg) of the SPG extract (SPG-247) was dissolved in a 1 M sodium hydroxide aqueous solution at a concentration of 1 mg/mL and gently shaken at 5°C for 24 hr. Thereafter, the mixture was filtered through an Omnipore membrane filter with a pore size of 5 µm and then the filter with a 1 µm pore size, and a 2-fold amount of methanol was added to the obtained filtrate to precipitate SPG. The precipitated SPG was washed several times with a mixed solution of methanol:water=3:1. This washing was repeated until the solution became neutral, and further washing once each with methanol and acetone was performed. Finally, the precipitate was dried at 40°C to obtain mSPG (mSPG-19a: yield 125 mg).

### Example 4: Control of SPG molecular weight by alkali treatment

Using the SPG extract produced using the Schizophyllum commune strain F-82046 described in Example 2 as a starting material, mSPG-1b was produced in the same manner as for mSPG-1a. Using mSPG-1b obtained here or mSPG-1a as a starting material, SPG with a controlled molecular weight was produced as follows. The mSPG was dissolved in a 5 M sodium hydroxide aqueous solution at a concentration of 1 mg/mL. The solution was gently shaken at 28°C to 50°C for 8 hr to 72 hr. Methanol in a 2-fold amount of the shaked solution was added to precipitate SPG. The precipitated SPG was washed several times with a mixed solution of methanol:water=3:1. This washing was repeated until the solution became neutral, and further washing once each with methanol and acetone was performed. Finally, the precipitate was dried at 40°C to obtain purified SPG having a controlled molecular weight. The temperature and time were changed within these ranges to produce SPG with various molecule sizes.

The produced SPGs are shown in Table 2.

**[Table 2]**

| SPG with controlled molecular weight | | | |
|---|---|---|---|
| SPG lot | mSPG as starting material | alkali treatment conditions* | yield |
| SPG-70 | mSPG-1a | 40°C, 24hr | 37 mg |
| SPG-71 | mSPG-1a | 40°C, 48hr | 36 mg |
| SPG-72 | mSPG-1a | 40°C, 72 hr | 26 mg |
| SPG-73 | mSPG-1a | 28°C, 24hr | 47 mg |
| SPG-76 | mSPG-1a | 50°C, 24hr | 69 mg |
| SPG-88 | mSPG-1b | 40°C, 8hr | 41 mg |
| SPG-89 | mSPG-1b | 40°C, 16 hr | 41 mg |
| SPG-93 | mSPG-1b | 40°C, 48 hr | 32 mg |
| SPG-95 | mSPG-1b | 48°C, 40 hr | 104 mg |

| | | | |
|---|---|---|---|
| * 5 M sodium hydroxide aqueous solution was used as medium and mSPG concentration was 1 mg/mL. | | | |

In addition, it was confirmed that SPG with a controlled molecular weight can be produced by a shaking treatment under various conditions in the range of 17°C to 55°C for 48 hr to 96 hr using an aqueous solution of sodium hydroxide with a concentration of 1 M to 5 M.

### Example 5: Control of SPG molecular weight by high-pressure homogenizer

As a high-pressure homogenizer, a pressure homogenizer (Emulsiflex C-5 model manufactured by Avestin or Microfluidizer M-110EH model manufactured by POWREX CORPORATION) was used. An SPG extract obtained by culturing Schizophyllum commune strain F-82046 was dissolved in 30 mL of distilled water to a concentration of 1 mg/mL to 3 mg/mL and allowed to infiltrate. The SPG solution was added to the flow path of the pressure homogenizer, and the SPG solution was repeatedly homogenized while gradually increasing the emulsification pressure to a maximum emulsification pressure of 15,000 psi to 25,000 psi.

To the treated SPG solution was added an equal volume of methanol and the mixture was allowed to stand for several hours, and the deposited precipitate was collected by centrifugation. The precipitate was washed with methanol, infiltrated with a small amount of water and freeze-dried. In this way, SPG with a molecular weight controlled by a method that does not rely on alkali treatment could be produced (SPG-85: yield 44 mg, SPG-86: yield 49 mg, SPG-87: yield 38 mg).

### Experimental Example 2: Molecular weight measurement

The molecular weight of SPG was measured by the following procedure as a value converted to pullulan used as a standard. A 0.25 M sodium hydroxide aqueous solution was added to SPG as a test substance, and the mixture was stirred at 5°C for 1 hr to prepare a measurement sample. Pullulan standards (molecular weight standard, Shodex. respective molecular weights: 2350 kD, 1220 kD, 642 kD, 337 kD, 194 kD, and 107 kD) were dissolved in purified water and used as measurement samples. A differential refractometer (RI) was connected to a size exclusion chromatography device (SEC), and these test samples were analyzed (referred to as SEC-RI method). A molecular weight calibration curve was prepared from the retention times of the pullulan standards and the molecular weights of the samples were estimated. The analytical conditions for the SEC-RI method are shown below.

High-performance liquid chromatograph system (injector, pump, column heater, RI detector): A1200 (Agilent), mobile phase: about 10 mM sodium hydroxide aqueous solution (pH12), flow rate: 0.4 mL/min, column: Asahipak GS-2G 7B (Shodex), Asahipak GS-620HQ (Shodex), Asahipak GS-320HQ (Shodex) connected in series in this order, column temperature: 40°C, sample concentration: about 2 mg/mL, sample injection volume:100 µL, analysis time: 75 min.

The molecular weight calibration curve is shown in Fig. 3. It was confirmed that the molecular weight can be measured in the pullulan-converted molecular weight range of 107 kD to 2350 kD ("Log (molecular weight)" in the range of 5.03 to 6.37).

The molecular weights of SPG calculated from the above-mentioned molecular weight calibration curve and the retention times of the molecular weight measurement samples are shown in Table 3 and Table 4. It was found that the molecular weight of SPG can be controlled over a wide range of from about 50 kD to 1000 kD or more under alkali treatment conditions. In addition, for SPG (mSPG) before molecular weight control, the weight average molecular weight could not be calculated because a part of the peak exceeded the upper limit of the molecular weight of the molecular weight calibration curve. However, the present measurements confirmed that these mSPGs had larger molecular weights than molecular weight-controlled SPGs, i.e., giant molecular weights of generally greater than 1000 kD. On the other hand, in some molecular weight-controlled SPGs and purchased SPGs (SPG-95, SPG-72, SPG-76, and iSPG-1), some of the peaks did not reach the lower limit of the molecular weight in the molecular weight calibration curve. However, since the distribution was observed, the peak top molecular weight and weight average molecular weight were calculated for reference. It was confirmed that these SPGs have smaller molecular weights than other SPGs, i.e., generally less than 100 kD.

**[Table 3]**

| Molecular weight of SPG | | |
|---|---|---|
| SPG lot*1 | peak top molecular weight | weight average molecular weight |
| SPG-73 (28°C, 24 hr) | 1151 kD | 1179 kD |
| SPG-88 (40°C, 8 hr) | 840 kD | 8 78 kD |
| SPG-89 (40°C, 16 hr) | 676 kD | 631 kD |
| SPG-93 (40°C, 48 hr) | 204 kD | 225 kD |
| SPG-95 (48°C, 40 hr) | 54 kD∗2 | 66 kD∗2 |

| | | |
|---|---|---|
| *1 The values in parentheses indicate the treatment temperature and time with a 5 M sodium hydroxide aqueous solution for molecular weight control. *2 Even though a part of the peak did not reach the lower molecular weight limit of the molecular weight calibration curve, distribution was observed; thus, the peak top molecular weight and weight average molecular weight were calculated for reference. | | |

**[Table 4]**

| Molecular weight of SPG | | |
|---|---|---|
| SPG lot*1 | peak top molecular weight | weight average molecular weight |
| mSPG-1a | 1447 kD | -∗2 |
| mSPG-2a | 1367 kD | -∗2 |
| SPG-70 (40°C, 24 hr) | 526 kD | 489 kD |
| SPG-71 (40°C, 48 hr) | 182 kD | 204 kD |
| SPG-72 (40°C, 72 hr) | 99 kD∗3 | 121 kD∗3 |
| SPG-76 (50°C, 24 hr) | 81 kD∗3 | 100 kD∗3 |
| iSPG-1 | -∗4 | -∗4 |

| | | |
|---|---|---|
| *1 The values in parentheses indicate the treatment temperature and time with a 5 M sodium hydroxide aqueous solution for molecular weight control. *2 Weight average molecular weight could not be calculated because a part of the peak exceeded the molecular weight upper limit of the molecular weight calibration curve. *3 Even though a part of the peak did not reach the lower molecular weight limit of the molecular weight calibration curve, distribution was observed; thus, the peak top molecular weight and weight average molecular weight were calculated for reference. *4 Multiple peaks with peak top less than 100 kD were detected. | | |

### Example 6: Production of complex of β(1→3) glucan and nucleic acid

SPG (mSPG, iSPG or molecular weight-controlled SPG) was selected as β(1→3) glucan, K3-dA40 was selected as nucleic acid, and a complex of β(1→3) glucan and nucleic acid was prepared as follows. Several mg of SPG was weighed, dissolved in 0.25 M aqueous sodium hydroxide solution to 10 mg/mL, and allowed to stand at 4°C for 24 hr with occasional stirring. Distilled water was added to K3-dA40 (59 sodium salt) to prepare a 100 µM aqueous solution. The above-mentioned SPG alkaline solution was added to the K3-dA40 aqueous solution at a predetermined ratio so that the mG/dA ratio was mainly 2.5, and the mixture was stirred to complex SPG with K3-dA40. Thereafter, 330 mM sodium dihydrogen phosphate aqueous solution in the same amount as the SPG alkaline solution was added for neutralization, and the mixture was allowed to stand overnight at 4°C to obtain K3-SPG.

As the SPG, those shown in Table 5 were used in addition to those shown in Example 2 or Table 2.

**[Table 5]**

| mSPG* used as starting material for K3-SPG | | |
|---|---|---|
| mSPG lot | Schizophyllum commune strain as derivation | yield |
| mSPG-3a | F-15516 | 15 mg |
| mSPG-4a | F-15567 | 11 mg |
| mSPG-5a | F-15977 | 28 mg |
| mSPG-8a | F-33504 | 33 mg |
| mSPG-9a | F-19065 | 17 mg |

| | | |
|---|---|---|
| *These mSPGs were produced by the method of Example 1. In addition, mSPG-1a, mSPG-1b, and SPG shown in Table 2 were used as starting materials for K3-SPG in this Example. | | |

K3-SPGs thus produced are shown in Table 6.

**[Table 6]**

| | | |
|---|---|---|
| Production example of complex consisting of β-glucan and nucleic acid | | |

| complex name | Lot No. | constituent SPG*1 |
|---|---|---|
| KAInv3701 | 20170613-01 | iSPG-1 (100or less) |
| KAmSP001a | 20170613-02 | mSPG-1a (huge) |
| KAmSP002a | 20170613-03 | mSPG-1a (huge) |
| KASPG0070 | 20170613-04 | SPG-70 (489) |
| KASPG0071 | 20170613-05 | SPG-71 (204) |
| KASPG0072 | 20170613-06 | SPG-72 (121∗2) |
| KASPG0076 | 20170613-07 | SPG-76 (100∗2) |
| KAInV3701 | 20170926-01 | iSPG-1 (100 or less) |
| KAmSP001b | 20170926-02 | mSPG-1a (huge) |
| KASPG0073 | 20170926-03 | SPG-73 (1179) |
| KASPG0088 | 20170926-04 | SPG-88 (878) |
| KASPG0089 | 20170926-05 | SPG-89 (631) |
| KASPG0093 | 20170926-06 | SPG-93 (225) |
| KASPG0095 | 20170926-07 | SPG-95 (66∗2) |
| KAmSP001a | 20171220-01 | mSPG-1a (huge) |
| KAmSP002a | 20171220-01 | mSPG-2a (huge) |
| KAmSP003a | 20171220-01 | mSPG-3a (huge) |
| KAmSP004a | 20171220-01 | mSPG-4a (huge) |
| KAmSP005a | 20171220-01 | mSPG-5a (huge) |
| KAmSP008a | 20171220-01 | mSPG-8a (huge) |
| KAmSP009a | 20171220-07 | mSPG-9a (huge) |
| KASPG0093 | 20171220-08 | SPG-93 (225) |
| KASPG0095 | 20171220-09 | SPG-95 (66∗2) |

| | | |
|---|---|---|
| *1 The weight average molecular weight of SPG in kD unit is shown in parentheses. For iSPG, since a plurality of peaks of 100 kD or less were observed, it is indicated as "100 or less". For SPG (mSPG) without control of molecular weight, all were 1000 kD or more and could not be measured numerically; thus they are indicated as "huge". *2 Even though a part of the peak did not reach the lower molecular weight limit of the molecular weight calibration curve, distribution was observed; thus, the peak top molecular weight and weight average molecular weight were calculated for reference. | | |

### Experimental Example 3: Evaluation of β(1→3) glucan and nucleic acid complex formation efficiency

The efficiency of forming a complex of the β(1→3) glucan produced in Example 6 and the nucleic acid was evaluated by SEC. As a measurement sample, the K3-SPG produced and obtained as a solution in Example 6 was used. In addition, the aqueous solution of K3-dA40 alone analyzed as a control was produced by the steps shown in Example 6 and using a 0.25 M sodium hydroxide aqueous solution that does not dissolve SPG so that it would have the same theoretical concentration of total K3-dA40 in the complex sample. The conditions for SEC are shown below.
High-performance liquid chromatography system (injector, pump, column heater, UV detector): A1100 (Agilent), mobile phase: 100 mM phosphate buffer (pH 7.4, nacalai tesuque, catalog No.: 37244-35), flow rate: 0.5 mL/min, column: Asahipak GF-1G 7B (Shodex), Asahipak GF-7 M HQ (Shodex), Asahipak GF-1G 7 M HQ (Shodex) connected in series in this order, column temperature: 40°C, detection wavelength: 260 nm, sample injection volume: 10 µL.

The efficiency of complexing was calculated from the remaining amount of K3-dA40 after complexing with respect to the amount of K3-dA40 before complexing. When all K3-dA40 has been complexed and the remaining amount is 0, the complex formation efficiency is 100%.

The measurement results of efficiency of the complex formation of K3-SPG produced in Example 6 are shown in Table 7. The formation efficiency is 96% or more for all, and it was confirmed that all purified SPG (before molecular weight control), SPG with controlled molecular weight, and purchased SPG form complexes with nucleic acids with extremely high efficiency by the production method of K3-SPG of the present invention.

**[Table 7]**

| Complex formation efficiency of K3-SPG | | |
|---|---|---|
| Complex name | lot No. | complex formation efficiency |
| KAInv3701 | 20170613-01 | 100% |
| KAmSP001a | 20170613-02 | 96% |
| KAmSP002a | 20170613-03 | 100% |
| KASPG0070 | 20170613-04 | 100% |
| KASPG0071 | 20170613-05 | 100% |
| KASPG0072 | 20170613-06 | 100% |
| KASPG0076 | 20170613-07 | 100% |
| KAInv3701 | 20170926-01 | 100% |
| KAmSP001b | 20170926-02 | 100% |
| KASPG0073 | 20170926-03 | 100% |
| KASPG0088 | 20170926-04 | 100% |
| KASPG0089 | 20170926-05 | 100% |
| KASPG0093 | 20170926-06 | 100% |
| KASPG0095 | 20170926-07 | 100% |
| KAmSP001a | 20171220-01 | 97% |
| KAmSP002a | 20171220-01 | 100% |
| KAmSP003a | 20171220-01 | 100% |
| KAmSP004a | 20171220-01 | 100% |
| KAmSP005a | 20171220-01 | 100% |
| KAmSP008a | 20171220-01 | 100% |
| KAmSP009a | 20171220-07 | 98% |
| KASPG0093 | 20171220-08 | 100% |
| KASPG0095 | 20171220-09 | 100% |

### Experimental Example 4: Evaluation of complex particle size by DLS

The particle size of the complex (K3-SPG) produced in Example 6 was measured as the average particle size based on the scattered light intensity by the photon correlation method in the DLS method. In this case, dilution with a phosphate buffer (8 g/L NaCl, 200 mg/L potassium dihydrogen phosphate, 1150 mg/L anhydrous phosphoric acid disodium hydrogen) was performed as appropriate to achieve the minimum K3-SPG concentration at which a detectable scattered light intensity was obtained.

The measurement results of the average particle size of K3-SPG produced in Example 6 are shown in Table 8. It was confirmed that the average particle size was strictly controlled within the range of 21.9 nm to 122.2 nm.

**[Table 8]**

| Particle size of K3-SPG | | |
|---|---|---|
| Complex name | lot No. | particle size |
| KAInv3701 | 20170613-01 | 31. 3 nm |
| KAmSP001a | 20170613-02 | 121. 1 nm |
| KAmSP002a | 20170613-03 | 122. 2 nm |
| KASPG0070 | 20170613-04 | 85. 1 nm |
| KASPG0071 | 20170613-05 | 44. 9 nm |
| KASPG0072 | 20170613-06 | 32. 1 nm |
| KASPG0076 | 20170613-07 | 29.5 nm |
| KAInv3701 | 20170926-01 | 30. 4 nm |
| KAmSP001b | 20170926-02 | 117, 3 nm |
| KASPG0073 | 20170926-03 | 104. 8 nm |
| KASPG0088 | 20170926-04 | 92.2 nm |
| KASPG0089 | 20170926-05 | 86. 4 nm |
| KASPG0093 | 20170926-06 | 51. 8 nm |
| KASPG0095 | 20170926-07 | 21. 9 nm |
| KAmSP001a | 20171220-01 | 105. 8 nm |
| KAmSP002a | 20171220-01 | 99. 0 nm |
| KAmSP003a | 20171220-01 | 102.9 nm |
| KAmSP004a | 20171220-01 | 102.3 nm |
| KAmSP005a | 20171220-01 | 102.8 nm |
| KAmSP008a | 20171220-01 | 104. 5 nm |
| KAmSP009a | 20171220-07 | 103.2 nm |
| KASPG0093 | 20171220-08 | 45. 7 nm |
| KASPG0095 | 20171220-09 | 21. 9 nm |

### Experimental Example 5: In vitro evaluation of the pharmacological activity of K3-SPG

Using human PBMCs (Lonza, catalog No.: Cat#CC-2702 Lot#31468) and spleen cells prepared from C57BL/6 mice, IFN-a production induction potency and IFN-γ production induction potency of each were evaluated. Human PBMCs and mouse spleen cells were seeded in 96-well plates at 1×10⁶ cells per well. K3-SPG was added to the cells at the K3-dA40 (59 sodium salt)-converted concentrations of 0, 0.75, 2, 6, 20 µg/mL, and hIFN-α in the culture supernatant after 24 hr was measured by cytokine ELISA kit (PBL). The hIFN-α induction potency of K3-SPG was evaluated by calculating the area under the curve (hereinafter to be referred to as AUC) of the hIFN-α induction level at all K3-SPG concentrations. Similarly, the IFN-γ induction potency of K3-SPG was evaluated. The results thereof are shown in Fig. 4. As shown in the Table on the right side of Fig. 4, KAmSP001a (average particle size: 121.1 nm) and KAmSP002a (average particle size: 122.2 nm) have large particle sizes, and KAInv3701 (average particle size: 31.3 nm) and KASPG0095 (average particle size: 21.9 nm) have small particle sizes. As shown in Fig. 4, in both human frozen PBMCs and mouse spleen cells, K3-SPG with a large particle size showed higher IFN-α and IFN-γ production induction potency as compared with K3-SPG with a small particle size.

### Experimental Example 6: In vitro evaluation of pharmacological activity of complex

The correlation between the IFN-α (hIFN-α) production level from human PBMCs (Lonza, Cat# CC-2702 Lot# 31468) treated with K3-SPG complex and the average particle size of K3-SPG was evaluated. Human PBMCs were seeded in 96-well plates at 1×10⁶ cells per well. Each BG-CpG was added to the cells at the CpG ODN concentrations of 0, 0.75, 2, 6, 20 µg/mL, and hIFN-α production level in the culture supernatant after 24 hr was measured by cytokine ELISA kit (PBL). The hIFN-α induction potency of BG-CpG was evaluated by calculating the AUC curve of the hIFN-α induction level at all CpG concentrations. The correlation between the hIFN-α production level and the average particle size was examined using the Spearman test. As a result, the correlation coefficient was 0.7789, indicating a positive correlation (Fig. 5). That is, it was confirmed that a larger particle size shows a stronger pharmacological activity within the average particle size range of 20 nm to 130 nm.

### Experimental Example 7: In vivo evaluation of pharmacological activity of K3-SPG

C57BL/6 mice were subcutaneously inoculated with 2×10⁵ B16 cancer cells, which is a melanoma cell line derived from C57BL/6 mice (day 0), and on day 9, K3 (GeneDesign, Inc.) or K3-SPG (complex name: KAmSP001a) produced in Example 3 was intravenously administered at amounts of 30 µg/head and 100 µg/head. Administration of K3 or K3-SPG was performed every other day and 3 times in total, and tumor size was measured over time. As a result, as shown in Fig. 6, a remarkable antitumor effect was observed in the group administered with K3-SPG (triangle) as compared with the group administered with K3 (square). That is, when the dosage was converted to the number of moles, the efficacy of K3-SPG, which had a weight difference of about three times as compared with K3, was superior to that of K3.

### Example 7: Production of BG-CpG in which β-glucan is lentinan

Lentinan was produced according to a conventional method, as described in the above-mentioned Description of Embodiments. Here, mushroom bed cultivation was applied as a method for cultivating shiitake mushroom fruiting body, and purification was performed by selecting and combining known purification means. Furthermore, the molecular weight thereof was controlled and purification was performed by the method shown in Example 4. Using the thus-obtained lentinan, BG-CpG was produced by the method shown in Example 6. BG-CpG containing lentinan as a constituent component is hereinafter denoted as K3-LNT. The average particle size of the produced K3-LNT is shown in Table 9. Table 9 also indicates the molecular weight control conditions for lentinan used for the production of each K3-LNT. It was confirmed that the particle size of K3-LNT can be controlled by changing the time of alkali treatment under predetermined conditions for lentinan. As is clear from Fig. 7 showing the relationship between the alkali treatment time of lentinan and the average particle size of K3-LNT, it was found that the present invention produces K3-LNT whose average particle size is strictly controlled within the range of 40 nm to 180 nm.

**[Table 9]**

| Particle size of K3-LNT | | | |
|---|---|---|---|
| Complex name | lot No. | molecular weight control conditions for lentinan | average particle size |
| KALNT0707 | 20201106-1 | (no molecular weight control) | 192. 5 nm |
| KALNT0709 | 20201106-03 | 5M NaOH, 48°C, 5 hr | 149. 2 nm |
| KA LNT0710 | 20201106- 04 | 5 M NaOH,48°C, 10 hr | 1 7 4 . 9 nm |
| KALNT07l2 | 20201106-06 | 5M NaOH, 48°C, 20 hr | 1 3 4. 9 nm |
| KALNT0706 | 20201106-07 | 5 M NaOH, 48°C, 38 20 hr | 69. 9 nm |
| KALNT0703 | 20201124-02 | 5M NaOH, 48°C, 40 20 hr | 6 0. 6 nm |
| KALNT0737 | 20201124-03 | 5M NaOH, 48°C, 64 hr | 5 7. 3 nm |
| KALNT0738 | 20201124-04 | 5 M NaOH, 48°C, 80 20 hr | 42. 5 nm |

### Experimental Example 8: In vitro evaluation of pharmacological activity of K3-LNT

In the same manner as in the above-mentioned Experimental Example 5, the IFN-γ production induction potency of K3-LNT was evaluated. Mouse spleen cells were selected as the cell type, and the number of cells seeded per well of a 96-well plate was 1×10⁷. The amount of K3-LNT added was two levels of 2 µg/mL and 10 µg/mL, and the amounts of IFN-γ production for each level after 24 hr in culture was measured. A total value thereof was used as an index of the strength of the pharmacological activity of K3-LNT. As a result, as shown in Fig. 8, the correlation coefficient was 0.9081 in the range of average particle size from about 40 nm to 180 nm, and it was confirmed that a larger particle size of K3-LNT shows a stronger pharmacological activity.

### Experimental Example 9: In vivo evaluation of the pharmacological activity of K3-LNT

In the same manner as in the above-mentioned Experimental Example 7, the antitumor effect of K3-LNT was evaluated. On day 0 of evaluation, 2.5×10⁵ or 5.0×10⁵ B16 cancer cells were transplanted subcutaneously into C57BL/6 mice, and a total of three doses of K3-LNT were administered every other day from day 7. The administration route of K3-LNT was intratumoral, and the dose was 50 µg/head. The dose of K3, which is a nucleic acid for comparison, was 16.7 µg/head. As shown in Fig. 9, it was confirmed that K3-LNT with an average particle size of 70 nm to 175 nm has a strong antitumor effect. In addition, when some samples were compared with K3 in the same manner as in Experimental Example 7, as shown in Fig. 10, K3-LNT with an average particle size of 149 nm or 175 nm was superior to K3.

### [Industrial Applicability]

The optimal particle size of BG-CpG was selected as a component of a therapeutic or prophylactic drug for cancer immunotherapy. The BG-CpG of the present invention has an average particle size strictly controlled within the range of 20 nm to 180 nm, preferably 20 nm to 130 nm, or 40 nm to 180 nm, more preferably 80 nm to 130 nm, or 130 nm to 180 nm. Furthermore, since the BG-CpG of the present invention can obtain a remarkable effect at a lower dose than conventional BG-CpG, it can contribute to the industrial benefits by being applied to diseases that are difficult to treat or prevent with conventional BG-CpG or produced at a lower cost than conventional BG-CpG.

This application is based on a patent application No. 2020-189032 filed in Japan (filing date: November 12, 2020), the contents of which are incorporated in full herein by reference.

## Claims

1. A complex of β(1→3) glucan and a nucleic acid, having a controlled particle size.

2. The complex according to claim 1, wherein the average particle size is 80 nm to 130 nm.

3. The complex according to claim 1, wherein the average particle size is 130 nm to 180 nm.

4. The complex according to claim 1 or 2, wherein the aforementioned β(1→3) glucan is schizophyllan.

5. The complex according to claim 1 or 3, wherein the aforementioned β(1→3) glucan is lentinan.

6. The complex according to any one of claims 1 to 5,
wherein the aforementioned nucleic acid is an oligonucleotide comprising a Type K CpG oligonucleotide.

7. The complex according to any one of claims 1 to 6,
wherein the aforementioned nucleic acid is a nucleic acid with a polydeoxyadenosine linked to the 3'-end of a Type K CpG oligonucleotide.

8. The complex according to any one of claims 1 to 7,
wherein the aforementioned nucleic acid is an oligodeoxynucleotide comprising a humanized Type K CpG oligodeoxynucleotide consisting of the nucleotide sequence shown in SEQ ID NO: 1 and a polydeoxyadenylic acid, the polydeoxyadenosine is linked to the 3'-end of the humanized Type K CpG oligodeoxynucleotide, and phosphodiester bonds in the oligodeoxynucleotide are partly or entirely substituted by phosphorothioate bonds.

9. A pharmaceutical composition comprising the complex according to any one of claims 1 to 8.

10. The pharmaceutical composition according to claim 9, which is for the prophylaxis or treatment of a viral infectious disease, cancer, an allergic disease, or intracellular parasitic protozoan or bacterial infection.

11. The pharmaceutical composition according to claim 10, which is for the prophylaxis or treatment of a viral infectious disease.

12. The pharmaceutical composition according to claim 11, wherein the viral infectious disease is an RS virus or influenza virus infectious disease.

13. An immunostimulating agent comprising the complex according to any one of claims 1 to 8.

14. The immunostimulating agent according to claim 13, which is a vaccine adjuvant.

15. A pharmaceutical composition comprising
(a) the complex according to any one of claims 1 to 8 or the immunostimulating agent according to claim 13 or 14, and
(b) an antigen.

16. The composition according to claim 15, which is for inducing an immune response to the antigen.

17. The composition according to claim 16, wherein the antigen is derived from a pathogen.

18. The composition according to claim 17, which is for the prophylaxis or treatment of a pathogen infectious disease.

19. The composition according to claim 18, wherein the pathogen is a virus.

20. The composition according to claim 19, wherein the virus is an RS virus or an influenza virus.

21. The composition according to claim 16, wherein the antigen is derived from cancer.

22. The composition according to claim 21, which is for the prophylaxis or treatment of cancer.

23. A pharmaceutical composition comprising
(a) the complex according to any one of claims 1 to 8, or the pharmaceutical composition according to any one of claims 9, 10, 15, 16, 21 and 22, and
(b) an anticancer agent.

24. The composition according to claim 23, wherein the anticancer agent is an immune checkpoint inhibitor.

25. The composition according to claim 24, wherein the immune checkpoint inhibitor is any of a PD1 inhibitor, a PDL-1 inhibitor, and a CTLA-4 inhibitor.

26. A method for producing the complex according to any one of claims 1 to 8, utilizing the molecular weight property of β(1→3) glucan.

27. A method for treating or preventing viral infectious disease, cancer, an allergic disease, or intracellular parasitic protozoan or bacterial infection, comprising administering an effective amount of a pharmaceutical composition comprising the pharmaceutical composition according to claim 9 to a test subject.

28. The method according to claim 27, wherein the viral infectious disease is an RS virus or influenza virus infectious disease.

29. A method for treating or preventing viral infectious disease, cancer, an allergic disease, or intracellular parasitic protozoan or bacterial infection, comprising administering an effective amount of a pharmaceutical composition comprising the pharmaceutical composition according to claim 15 to a test subject.

30. The method according to claim 29, wherein the viral infectious disease is an RS virus or influenza virus infectious disease.

31. The method according to claim 27 or 29 which is used in combination with other anticancer agent.

32. The method according to claim 31, wherein the anticancer agent is an immune checkpoint inhibitor.

33. The method according to claim 32, wherein the immune checkpoint inhibitor is any of a PD1 inhibitor, a PDL-1 inhibitor, and a CTLA-4 inhibitor.
